(19) Europäisches Patentamt
European Patent Office
Office européen des brevets

(11) **EP 0 799 016 B1**

(12) **EUROPEAN PATENT SPECIFICATION**

(45) Date of publication and mention of the grant of the patent:
**04.12.2002 Bulletin 2002/49**

(21) Application number: **95943141.2**

(22) Date of filing: **18.12.1995**

(51) Int Cl.$^7$: **A61K 6/083**

(86) International application number:
**PCT/US95/16527**

(87) International publication number:
**WO 96/019179 (27.06.1996 Gazette 1996/29)**

# (54) POLYMERIZABLE COMPOUNDS AND COMPOSITIONS

POLYMERISIERBARE VERBINDUNGEN UND ZUSAMMENSETZUNGEN

COMPOSES ET COMPOSITIONS DE POLYMERISATION

(84) Designated Contracting States:
**CH DE FR GB LI**

(30) Priority: **19.12.1994 US 359217**

(43) Date of publication of application:
**08.10.1997 Bulletin 1997/41**

(73) Proprietor: **DENTSPLY INTERNATIONAL, INC.**
**York Pennsylvania 17405 (US)**

(72) Inventors:
- **KLEE, Joachim, E.**
  **D-79315 Radolfzell (DE)**
- **WALZ, Uwe**
  **D-78465 Konstanz (DE)**

(74) Representative:
**Wächtershäuser, Günter, Prof. Dr.**
**Patentanwalt,**
**Tal 29**
**80331 München (DE)**

(56) References cited:

| | |
|---|---|
| **EP-A- 0 212 193** | **EP-A- 0 219 058** |
| **EP-A- 0 356 868** | **DD-A- 208 365** |
| **DE-A- 4 109 048** | **DE-A- 4 217 761** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The invention relates to polymerizable macromonomers and dental and medical compositions containing polymerizable macromonomers. The invention provides macromonomers for dental compositions and a process for preparing them. Dental/medical compositions which include macromonomers of the invention have a high adhesion to hard dental tissue and low volumetric shrinkage.

It. is an object of the invention to provide an esterified macromonomer within the scope of the general formula:

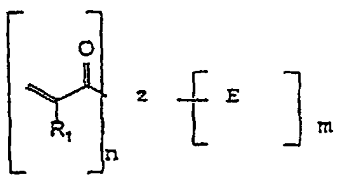

wherein Z is an organic moiety; $R_1$ is hydrogen or a substituted or unsubstituted alkyl having from 1 to 12 carbon atoms, oxyalkyl having from 1 to 12 carbon atoms, alkenyl having from 2 to 12 carbon atoms; cycloalkyl having from 5 to 12 carbon atoms, aryl having from 6 to 12 carbon atoms or aralkyl having from 7 to 12 carbon atoms, each E independently is a hydroxyl group or an organic ester moiety or an inorganic ester moiety and at least one E is an organic or inorganic ester moiety, n and m each independently is an integer from 2 to 12.

It is the object of the invention that the above esterified macromonomer is obtainable by esterification of at least a portion of the -OH groups of an -OH group containing macromonomer having at least one terminal double bond with at least one derivative of an inorganic or organic acid which introduces pendant groups exhibiting at least one acid moiety selected from the group of consisting of -COOH, $-PO_3H_2$, $-SO_3H$, $-BO_2H$ and salts thereof. The number of the acid moieties is chosen such that a polymer obtained by polymerizing said monomers has an adhesive strength to dentine of at least 2 MPa.

Prior Art dental/medical compositions such as cements are either water-based ionic cements or resin based materials. The water-based cements have the advantage of a modest adhesion to hard tooth tissues and of a high fluoride. ion release from inorganic filler material. They have the disadvantage of high water solubility, low abrasion resistance and an excessive opacity. The resin-based materials have the advantage of excellent mechanical properties, a suitable opacity and low water solubility. They have the disadvantage of a lack of adhesion, a very poor release of fluoride ions from an inorganic filler and a high volumetric shrinkage.

Engelbrecht et al in U.S. Patent 4,806,381 discloses Polymerizable Compounds Containing Acid and Acid. Derivatives, Mixtures Containing the Same, and Use Thereof. Blackwell et al in U.S. Patent 4.816.495 discloses Biologically Compatible Adhesive Visible Light Curable Compositions.

DE-A-41 09 048 discloses polymerizable addition products of epoxide oligomers and (meth)acrylic acid.

EP-A-0 219 058 discloses polymerizable cement compositions comprising polymerizable unsaturated monomers and/or oligomers, fillers and curing agents.

EP-A-0 356 868 discloses a method of treating a tooth comprising coating the tooth with a resin base composition which includes an adhesive.

The disadvantages of prior art dental compounds and compositions are overcome by the novel and nonobvious compounds and compositions of the invention.

BRIEF DESCRIPTION OF THE INVENTION

An esterified macromonomer obtainable by esterification of at least a portion of -OH groups of an OH- group containing macromonomer having at least one terminal double bond with at least one derivative of an inorganic or organic acid whereby pendant groups are introduced exhibiting at least one acid moiety selected from the group consisting of -COOH, $-PO_3H_2$, $-SO_3H$, $- BO_2H$ or salts thereof, whereby the esterified macromonomer is within the scope of the general formula:

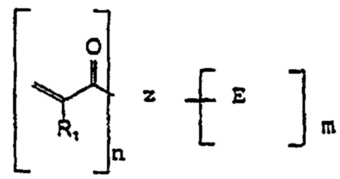

wherein Z is an organic moiety,

$R_1$ is hydrogen or a substituted or unsubstituted alkyl having from 1 to 12 carbon atoms, oxyalkyl having from 1 to 12 carbon atoms, alkenyl having from 2 to 12 carbon atoms, cycloalkyl having from 5 to 12 carbon atoms, aryl having from 6 to 12 carbon atoms or aralkyl having from 7 to 12 carbon atoms,

each E independently is a hydroxyl group or an organic or inorganic ester moiety and at least one E is an organic or inorganic ester moiety,

n and m each independently is an integer from 2 to 12.

The number of the acid moieties is chosen such that a polmer obtained by polymerizing those monomers has an adhesive strength to dentine of at least 2 MPa.

***DETAILED DESCRIPTION OF THE INVENTION***

The invention provides macromonomers esterfied, with organic acids or inorganic acids or derivatives thereof. The esterified macromonomers are useful in composition with or without water, such as water free self-adhesive dental/ medical composite. The dental/medical composite comprises a modified macromonomer, and/or di-or poly(methacrylates) containing phosphoric acid ester groups or salts thereof, polymerizable monomers, acid-reactive and/or reactive and/or non-reactive fillers, diluents, polymerization initiators and stabilizers. Composition in accordance with the invention include polymerization initiators, such as thermal initiators, redox initiators and/or photoinitiators. The new adhesive dental composite develops adhesion to dentine of about 4 MPa. Fillers of high X-ray absorbence provide radio-opacity values greater than that of the same thickness of aluminium.

***Preparation of epoxide-macromonomers***

Macromonomers in accordance with the invention are produced by chemical modification of macromonomers containing hydroxyl groups. Macromonomer containing hydroxyl groups useful for making esterified macromonomer in accordance with the invention are described for example in Polym. Bull. **27** (1992) 511-517, Acta Polym. **42** (1991) 17-20 and DE 4217761.8 incorporated herein by reference. Preferred polymerizable compounds for use in compositions in accordance with the invention are within the scope of general formulas M1-M12 as follows:

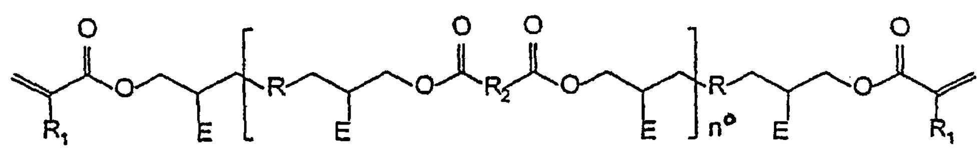

**M-1**

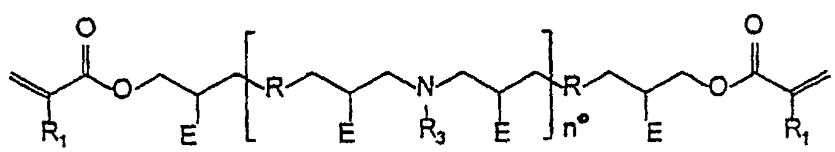

**M-2**

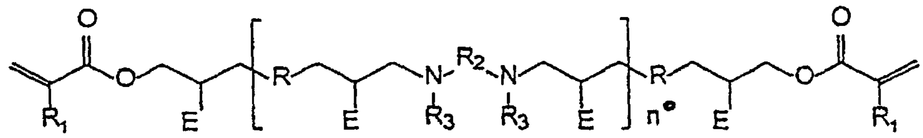

M-3

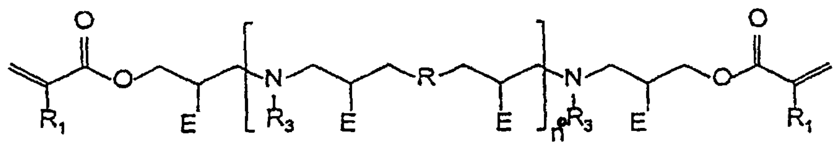

M-4

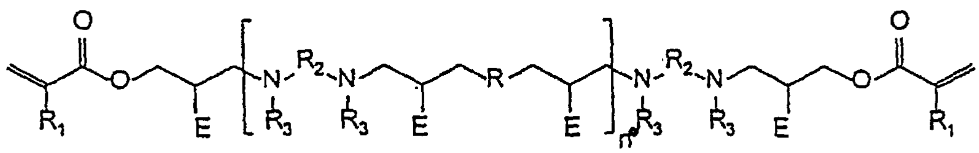

M-5

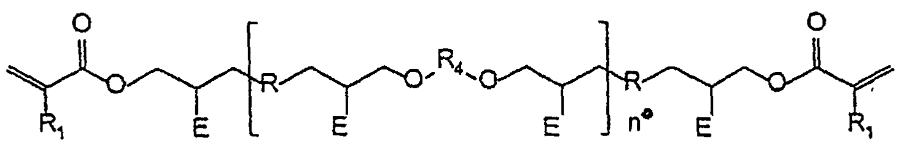

M-6

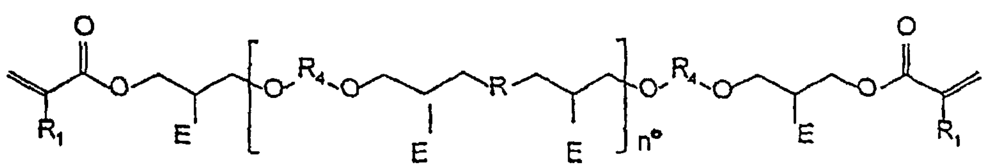

M-7

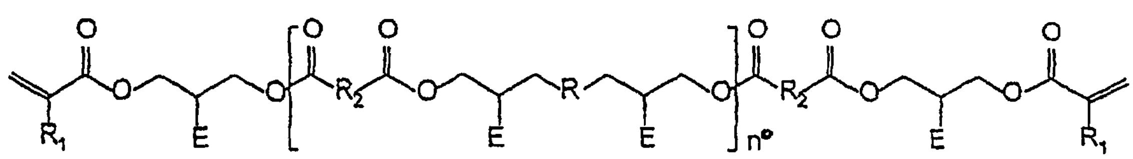

M-8

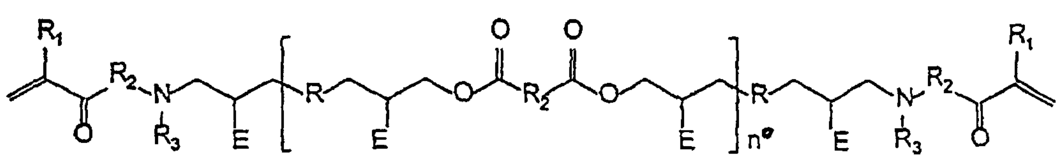

M-9

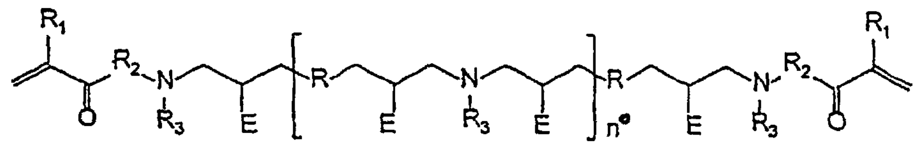

M-10

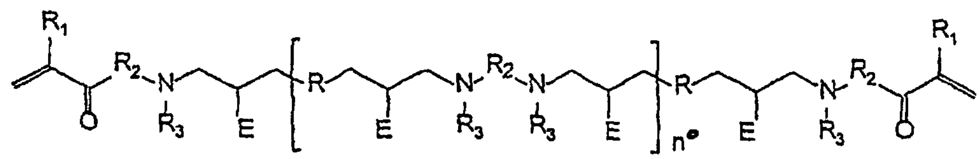

M-11

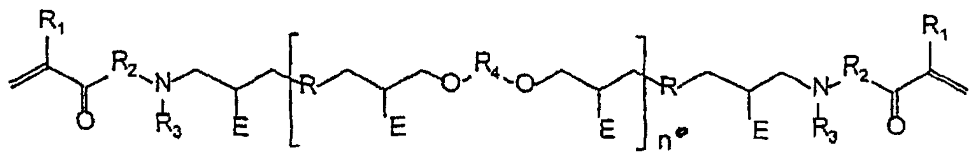

M-12

wherein
each E independently is a hydroxyl group, an organic ester moiety or an inorganic ester moiety,
at least one E is an ester moiety,
R is a diether or a diester containing moiety or tertiary amine,
$R_1$ is hydrogen or a substituted or unsubstituted alkyl having from 1 to 12 carbon atoms, oxyalkyl having from 1 to 12 carbon atoms, alkenyl having from 2 to 12 carbon atoms, cycloalkyl having from 5 to 12 carbon atoms, aryl having from 6 to 12 carbon atoms or aralkyl having from 7 to 12 carbon atoms,
$R_2$ is a difunctional substituted or unsubstituted alkyl group having from 1 to 12 carbon atoms, alkenyl group having from 2 to 12 carbon atoms, cycloalkyl having from 5 to 12 carbon atoms, aryl having from 6 to 12 carbon atoms or aralkyl having from 7 to 12 carbon atoms,
$R_3$ is hydrogen or a substituted or unsubstituted alkyl group having from 1 to 12 carbon atoms, alkenyl group having from 2 to 12 carbon atoms, cycloalkyl having from 5 to 12 carbon atoms, aryl having from 6 to 12 carbon atoms or aralkyl having from 7 to 12 carbon atoms,
$R_4$ is a substituted or unsubstituted aryl having from 6 to 12 carbon atoms,
and n° is an integer of at least 1.

Preferably R is a moiety within the scope of the general formulas:

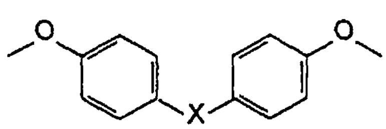 , 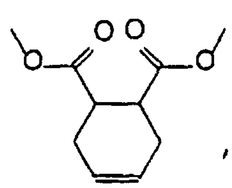 , 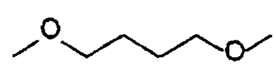 or 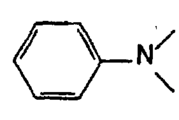

(according to R of the foreclosures)
wherein X is $C(CH_3)_2$, $-CH_2-$, -O-, -S-, -CO-, or $-SO_2-$.

Preferably $R_4$ is a moiety within the scope of the general formulas:

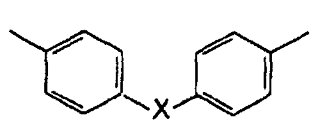 or 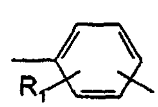

wherein X is $C(CH_3)_2$, $-CH_2-$, -O-, -S-, -CO-, $-SO_2-$.

Preferably E is a hydroxyl group, an ester moiety, a boric acid moiety, a sulfuric acid moiety or a phosphoric acid moiety.

Macromonomers within the scope of general formula **M-1** are synthesized in two steps. At first an oligomer mixture is obtained by reaction of an α,β-unsaturated acids with excessive amounts of a diepoxide, such as bisphenol-A diglycidyl ether (DGEBA), bisphenol-F diglycidyl ether (DGEBF), butanediol diglycidyl ether (BDODGE), tetrahydro terephtalic acid diglycidyl ether or diglycidyl aniline. This mixture contains the bis-ester of the diepoxide along with the mono-ester and unreacted diepoxide as governed by the ratio of the diepoxide and the unsaturated acid. The formation of macromonomers follows in a second reaction of the previous reacted oligomers with dicarboxylic acids to **M-1** (DE 4217761.8).

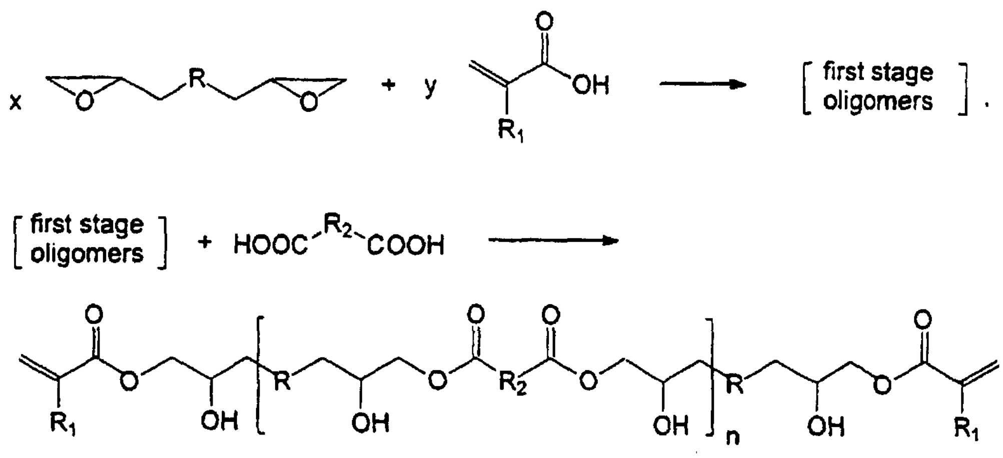


**M-1(wherein E is hydrogen)**

Instead of dicarboxylic acids in the second step also primary monoamines were used which react to macromonomers **M-2**, disecondary diamines which react to macromonomers **M-3,** (J.Klee et. al. Polym. Bull. **27** (1992) 511-517, DD 279667) and bisphenols which react to macromonomers **M-6**.

During the epoxide ring cleavage by carboxylic acids an amount equal to approximately 20 percent by weight of the epoxide groups is opened to the corresponding primary alcohols:

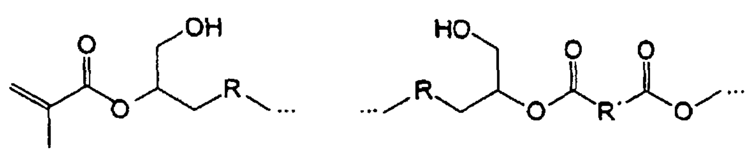

Consequently, macromonomers **M-1, M-2, M-3** and **M-6** wherein each E is hydrogen contain both types of molecules having primary and/or secondary alcohol units.

The resulting macromonomers are viscous liquids or solids which are soluble in THF, CHCl3 and DMF. Their glass transition temperatures are relatively low (between 0 and 50 °C) depending on the nature of the comonomer and the molecular mass of the macromonomers.

The degree of polymerisation $P_n$ and the macromonomer value n depends on the mol ratio of the monomers, the diepoxide and the comonomers and were calculated by

$$P_n = \frac{1+r}{1-r} \quad \text{and} \quad n = \frac{r}{1-r},$$

respectively using r=zlx. That means each macromonomer **M** is a definite mixture of a series of homologous oligomers (n=1,2,3,4,5,...) and contains a certain amount of the molecule (n=0).

Macromonomers **M-5** wherein each E is hydrogen are prepared by one-step reaction of the diepoxides, disecondary diamines and 2,3-epoxypropyl-(meth)acrylate according to the following equation:

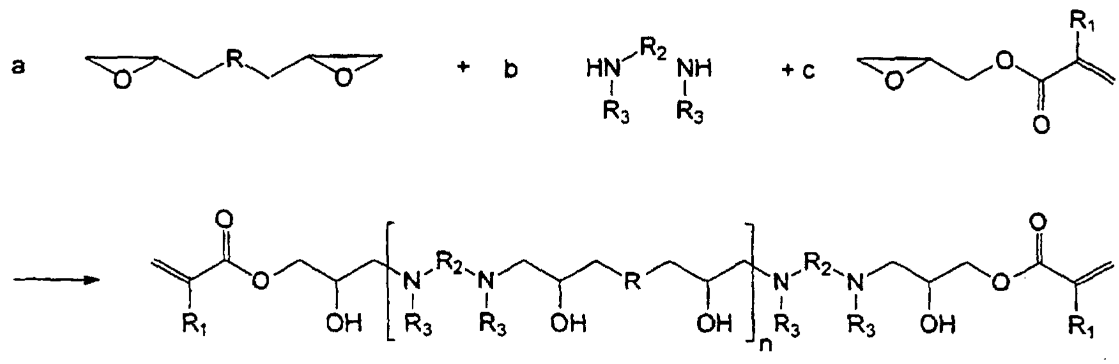

**M-5 (wherein E is hydrogen)**

A second route to obtain macromonomers **M-5** wherein each E is hydrogen is a two-step reaction. In the first step the diepoxide is reacted with the disecondary diamine to an α,ω-terminated prepolymer. In the second step the obtained prepolymer is reacted with 2,3-epoxypropyl-(meth) acrylate (DD 277689, J.Klee, H.-H. Hörhold, H.Schütz, Acta Polym. **42** (1991) 17-20).

Instead of disecondary diamines in the second step also were used primary monoamines react to macromonomers **M-4**, bisphenols react to macromonomers **M-7** or dicarboxylic acids react to macromonomers **M-8.**

Macromonomers **M-9** wherein each E is hydrogen are prepared by reaction of diepoxides, dicarboxylic acids and aminoalkyl (meth)acrylates according to the following equation:

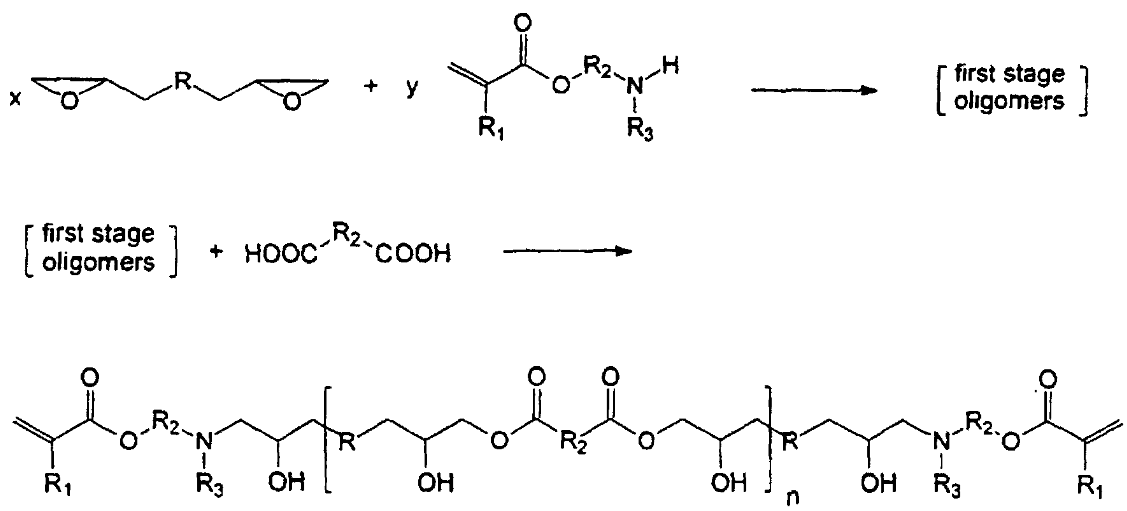


**M-9 (wherein E is hydrogen)**

Instead of dicarboxylic acids, primary monoamines were used to prepare macromonomers **M-10,** disecondary diamines were used to prepare macromonomers **M-11,** bis-phenols were used to prepare macromonomers **M-12.**

Specific macromonomers **M-1** to **M-12** representing molecules of n=0, n=1 or n=2 may be isolated from the mixture by fractionated precipitation or by chromatography and subjected to esterification as described.

***Esterification of macromonomers***

The reaction of epoxide macromonomers **M-1** to **M-12** with organic acids or inorganic acids or derivatives thereof leads to macromonomers having ester moieties.

As derivatives of organic acids preferably were used succinic acid anhydride, maleic acid anhydride, dichloromaleic acid anhydride, dimethyl maleic acid anhydride, ma-Ionic acid anhydride, aconit acid anhydride, adipic acid

anhydride, 3,3-tetramethylen glutaric acid anhydride, cyclohexen-1,2 acid anhydride, nadinic acid anhydride, phthalic acid anhydride, trimellitic acid anhydride, 2-sulfo-benzoic acid anhydride, 2-sulfo succinic acid anhydride, phthalic acid anhydride p-(O-phosphat), phthaloylchloride, succinic acid dimethyl ester.

As derivatives of inorganic acids preferably were used phosphorous penta chloride, phosphorous trichloride, phosphorous oxychloride, sutfuryl chloride, thionyl chloride, phosphor thionyl chloride, boric acid anhydride, boron trichloride.

It is possible to synthesize the esterified macromonomers without using any catalysts in the cases of **M-2** to **M-5, M-10, M11** (n>0). These macromonomers contain the catalytic active amine in the backbone of the molecule. The use of catalysts such as tertiary amines or quarterly ammonium salts is possible and in the case of esterification of **M-1, M-6, M-7, M-8, M-9** and **M-12** necessary.

The esterification of the macromonomer hydroxyl groups is carried out in pure substance or in diluted solutions. Preferably, solvents such as tetrahydro furane, dioxane, or polymerizable monomers such as triethylenglycol bismethacrylate, diethylenglycol bismethacrylate, dioxolan bismethacrylate, vinyl-, vinylen- or vinyliden-, acrylate- or methacrylate substituted spiroorthoesters and 2,2-Bis[p-(acryloxyethoxy)phenyl]propane are present during esterification of the macromonomers. The temperature is in the preferred range of 60 °C to 120 °C.

***Dental/medical application***

A dental/medical composite, a dental/medical sealant, a dental/medical adhesive and a dental/medical primer have been developed comprising a modified $\alpha,\omega$-(meth) acryloyl terminated macromonomer notably a di- or poly(meth) acrylate monomer having phosphorous ester groups or salts thereof, polymerizable monomers, fillers, polymerization initiators and stabilizers.

As di- or poly(meth)acrylate monomer having phosphorous ester groups and salts thereof are employed pentaerythrit triacrylate monophosphate, dipentaerythrit penta-acrylate monophosphate, glycerol di(meth)acrylate monophosphate, triethylenglycol (meth)acrylate monophosphate.

As organic polymerizable monomers were used mono- and polyfunctional (meth)-acrylates, such as polyalylenoxide di- and poly(meth)acrylates, urethane di- and poly(meth)acrylates, vinyl-, vinylen- or vinyliden-, acrylate- or methacrylate substituted spiroorthoesters, spiroorthocarbonates or bicyloorthoesters. Preferably were used diethylenglycol dimethacrylate, triethylenglycol dimethacrylate, 3,(4),8,(9)-dimethacryloyloxymethyltricyclodecane, dioxolan bismethacrylate, glycerol trimethacrylate, furfuryl methacrylate in a content of 5 to 80 wt-%.

As polymerization initiators are used thermal initiators, redox initiators and/or photo initiators in a content of 0,001 to 3 wt-%.

Thermal initiators are initiators such as peroxides, peresters, perketals, peroxy carbonates, hydroxyperoxides, persulfates and azo compounds preferably dibenzoyl peroxide, cumol hydroperoxide, diisopropyl peroxycarbonate, dipotassium persulfate, azobisisobutylonitril.

Preferred redox initiator systems for use in compositions in accordance with the invention are peroxide/amine systems, such as peracid/amine, perester/amine, perketal/amine, peroxycarbonatelamine and hydroxyperoxide/amine systems; peroxide/metal ion salts, such as ascorbic acid/peroxide/metal ion compounds, (thio)barbituric acid/peroxide/ metal ion compounds, metal ion compounds/sulfinates, metal ion compounds/(thio)barbituric acid; transition metal carbonyl compounds and halogenids of organic compounds; boralkyl compounds, peroxysulfates and thiols. Most preferred redox-initiators are benzoylperoxide/N,N-bis-($\beta$-hydroxyethyl)-p-toluidine, benzoylperoxide/N,N-bis-($\beta$-hydroxyethyl)-p-benzoic acid ethylester, benzoylperoxide/tributylamine, cumol hydroperoxide/N,N-bis-($\beta$-hydroxyethyl)-p-toluidine, diisopropyl peroxycarbonate/dimethylbenzylamine.

Preferred photoinitiators for use in polymerizable compositions in accordance with the invention which include macromonomers with the scope of general formulas M-1 through M-12 are camphorquinone, benzophenone and 2,2-dimethylbenzylketal.

Preferred fillers for use in compositions in accordance with the invention include inorganic compounds, such as $La_2O_3$, $ZrO_2$, $BiPO_4$, $CaWO_4$, $BaWO_4$, $SrF_2$, $Bi_2O_3$, glasses and/or organic fillers, such as polymer granulate. Dental/ medical composite compositions of the invention preferably include filler in an amount from about 50 to about 85 percent by weight. Dental/medical adhesive compositions of the invention preferably include filler in an amount from about 50 to about 65 percent by weight. Dental/medical sealant compositions of the invention preferably include filler in an amount from about 10 to about 50 percent by weight.

Dental/medical composite compositions, adhesives and sealant of the invention include one-component and two-component paste/paste and powder/ liquid-material which is to be mixed immediately before use.

Shrinkage of composite compositions of the invention is-preferably less than 4.5 and more preferably less than 1.5 percent by volume. Adhesive dental composite compositions of the invention containing radio-opaque fillers preferably provide a radio-opacity of at least 1.5 mm/ mm Al, more preferably at least 3 to 7 mm/mm Al, and most preferably at least 7 mm/mmAl.

The self-adhesive dental/medical composites compositions in accordance with a preferred embodiment of the invention have a fluoride release of at least 1 μg/cm$^2$, more preferably at least 1-3 μg/cm$^2$, and most preferably at least 3-10 μg/cm$^2$.

Self-adhesive dental/medical composites compositions in accordance with a preferred embodiment of the invention have an opacity of at least 40 %, more preferably at least 20-40 %, and most preferably at least 5-20 %.

The setting time of the adhesive dental/medical adhesive compositions in accordance with a preferred embodiment of the invention at 37°C is between 1 minute and 60 minutes, more preferably between 5 and 30 minutes and most preferably between 2 and 5 minutes. The setting time of adhesive compositions in accordance with a preferred embodiment of the invention at 23°C is preferably between 10 minutes and 300 minutes more, preferably between 5 and 100 minutes and most preferably between 5 and 20 minutes.

Dental/medical composition in accordance with the invention is characterised by having an adhesion to dentine of at least 2 MPa; a fluoride release of at least 1 μg F-per week and per cm$^2$ of the exposed surface of the composition; an opacity of at least $C_{0,7}$= 40 %; and a compressive strength of at least 200 Mpa.

In the following examples bond strength to dentin is measured using extracted human teeth. The teeth used for the shear bond strength test are treated in 1% sodium hypochlorite for one hour and then stored in distilled water in a refrigerator at about 4°C until needed. The teeth are washed with water, mechanically sanded with 320 grit carborundum paper until a flat dentin surface is exposed.

The teeth are then individually blown dry with compressed dry air to ensure the dentin surface is free from noticeable moisture. A small plastic straw with 5 mm inner diameter and 2 to 3 mm in length is filled with the polymerizable composition being tested and seated on the dentin so as to form a post without pressure. The upper open end of the straw is covered with a thin film and cured. The specimens are then stored in distilled water at 37°C for 24 hours. The teeth are then vertically mounted in a 7 cm ring using gypsum to provide a base for testing with the post at right angles thereto. The mounted specimens are then loaded in shear in an Zwick device model number 1455 manufactured by Zwick GmbH for measurement of adhesion of the post to dentin at 1 mm/minute crosshead speed. The load is applied parallel to the prepared tooth surface and at right angles to the post until fracture occurred. The shear bond strength is then calculated.

In the examples Fluoride Release is measured by making three 1 X 20 mm (diameter) discs of each material. Each disc is placed in 25 ml water stored for a week at 37°C. Using an ion selective electrode, the fluoride concentration in mg $F^-$/cm$^2$ is determined for each disc. The average value of the three discs is recorded.

In the Examples compressive strength is measured according to ISO 9917, EN 29917; flexural strength is measured according to ISO 4049, EN 24049; elastic modulus is measured according to ISO 4049, EN 24049; opacity is measured according to ISO 9912, EN 29912; IR spectra are measured using a Fourier transformation Infra Red spectrometer at 23°C.

## Reference Example 1

The macromonomer of formula M-1wherein n° is 1, R is $-OC_6H_4-C(CH_3)_2-C_6H_4O-$, $R_1$ is $-CH_3$, $R_2$ is $-(CH_2)_4-$ is referred to hereinafter as macromonomer M-1A and is prepared by reacting 150.000 g (0.441 mol) bisphenol-A diglycidyl ether, 32.200 g (0.220 mol) adipic acid and 2,000 g triethylbenzylammoniumchloride for four hours at 80 °C while stirring. To the obtained glycidyl terminated prepolymer are added 37.900 g (0.441 mol) methacrylic acid and 0.444 g 2.6-di-tent.-butyl-p-cresol and are reacted for another four hours at 80 °C. The methacrylate terminated macromonomer is soluble in organic solvents such as chloroform, DMF and THF. In the IR-spectrum no absorption of epoxide groups at v=915 and 3050 cm$^{-1}$ is observed. Absorption of ester groups is seen at v=1720 cm$^{-1}$. In the $^1$H NMR spectrum are found signals of the olefinic double bond at $\delta_{(CH2=)}$= 6,137 / 6,119/6.115 ppm and at $\delta_{(CH2=)}$= 5,587 / 5,582 /5,555 / 5,548 ppm.

## Reference Example 2

Preparation of the macromonomer of formula M-1B wherein E is hydroxyl, n° is 1, R is $-O(CH_2)_4O-$, $R_1$ is $-CH_3$, $R_2$ is $-(CH_2)_4-$.

200.00 g (0.99 mol) butanediol digiycidyl ether, 72.26 g (0.49 mol) adipic acid, 85.13 g (0.99 mol) methacrylic acid, 4.72 g triethylbenzylammoniumchloride and 0.60 g 2,6-di-tert.-butyl-p-cresol are stirred together and heated for four hours at 90 °C. The obtained methacrylate terminated macromonomer is soluble in organic solvents such as chloroform, DMF and THF. In the IR-spectrum no absorption of epoxide groups at 915 and 3050 cm$^{-1}$ is observed. Absorption of ester groups is seen at 1720cm$^{-1}$. The viscosity measured with a Bohlin rheometer is $\eta_{dyn}$ = 3.3 Pas (25°C).

**Reference Example 3**

Preparation of the macromonomer of formula M-1F wherein E is hydroxyl, n°is 1, R is $-OC_6H_4-CH_2-C_6H_4O-$, $R_1$ is $-CH_3$, $R_2$ is $-(CH_2)_4-$.

100.00 g (0,32 mol) bisphenol-F diglycidyl ether, 23.39 g (0.16 mol) adipic acid, 27.56 g (0.32 mol) methacrylic acid, 65.47 g triethylenglycol dimethacrylate, 1.53 g triethylbenzylammoniumchloride and 0.30 g 2,6-di-tert.-butyl-p-cresol are stirred together and heated for four hours at 90 °C. The obtained methacrylate terminated macromonomer is soluble in organic solvents such as chloroform, DMF and THF. In the IR-spectrum no absorption of epoxide groups at 915 and 3050 $cm^{-1}$ is observed.

Absorption of ester groups is seen at 1720 $cm^{-1}$. The viscosity measured with a Bohlin rheometer is $\eta_{dyn}$ = 3.6 Pas (25°C).

**Reference Example 4**

Preparation of the macromonomer of formula **M-3** wherein E is hydroxyl n°is 1, R is $-OC_6H_4-C(CH_3)_2-C_6H_4O-$, $R_1$ is $-CH_3$, $R_2$ is $-(CH_2)_4O(CH_2)_4-$, $R_3$ is $C_6H_5CH_2-$.

150.000 g (0,441 mol) bisphenol-A diglycidyl ether, 37.935 g (0.441 mol) methacrylic acid, 2.000 g triethylbenzylammonium chloride, 1.115 g 2,6-di-tert.-butyl-p-cresol (BHT) and 111,695 g triethylenglycol dimethacrylate were homogeneously mixed while heating. The mixture was kept for two hours at 90 °C. After this time 75.020 g (0.221 mol) N,N'-diberizyl-5-oxanonanediamine-1,9 were added to the mixture while stirring and kept for additional two hours at 90 °C. The obtained methacrylate terminated macromonomer is soluble in organic solvents such as chloroform, DMF and THF. No absorption of epoxide groups at 915 and 3050 $cm^{-1}$ is observed in the IR-spectrum. Absorption of ester groups were found at 1720 $cm^{-1}$.

**Reference Example 5**

Preparation of the macromonomer of formula **M-5** wherein E is hydroxyl, n°is 1, R is $-OC_6H_4-C(CH_3)_2-C_6H_4O-$, $R_1$ is $-CH_3$, $R_2$ is $-(CH_2)_4O(CH_2)_4-$, $R_3$ is $C_6H_5CH_2-$.

20.000 g (58.75 mmol) bisphenol-A diglycidyl ether and 40.012 g (117.50 mmol) N,N'-dibenzyl-5-oxanonanediamine-1,9 are homogeneously mixed while heating. The mixture is kept for two hours at 90 °C. After this time 16.704 g (117.50 mmol) 2,3-epoxypropyl methacrylate is added to the mixture while stirring and the mixture is for another two hours at 90 °C. The obtained methacrylate terminated macromonomer is soluble in organic solvents such as chloroform, DMF and THF. In the IR-spectrum no absorption of epoxide groups at 915 and 3050 $cm^{-1}$ is observed.

**Reference Example 6**

Preparation of the macromonomer of formula **M-5** wherein E is hydroxyl, n°is 0, $R_1$ is $-CH_3$, $R_2$ is $-(CH_2)_4O(CH_2)_4-$, $R_3$ is $C_6H_5CH_2-$.

50.000 g (146,83 mmol) N,N'-dibenzyl-5-oxanonanediamine-1.9, 41.750 g (293.67 mmol) 2,3-epoxypropyl methacrylate and 0.213 g BHT are homogeneously mixed while heating. The mixture is kept for two hours at 90 °C. The obtained methacrylate terminated macromonomer is soluble in organic solvents such as chloroform, DMF and THF. In the IR-spectrum no absorption of epoxide groups at 915 and 3050 $cm^{-1}$ are observed.

**Reference Example 7**

Preparation of the macromonomer of formula **M-6** wherein E is hydroxyl, n° is 1, R is $-OC_6H_4-C(CH_3)_2-C_6H_4O-$, $R_1$ is $-CH_3$, $R_4$ is $-C_6H_4-C(CH_3)_2-C_6H_4-$.

150.000 g (0.441 mol) bisphenol-A diglycidyl ether, 50.299 g (0.220 mol) 2,2-bis-(4-hydroxy-phenyl)propane, 37.901 g (0.441 mol) methacrylic acid, 102.086 g triethylenglycol dimethacrylate, 2.000 g triethylbenzylammoniumchloride and 0.959 g 2,6-di-tert.-butyl-p-cresol are heated for four hours at 80 °C.

The obtained methacrylate terminated macromonomer is soluble in organic solvents such as chloroform, DMF and THF. In the IR-spectrum no absorption of epoxide groups at 915 and 3050 $cm^{-1}$ is observed. Absorption of ester groups is found at 1720 $cm^{-1}$.

**Reference Example 8**

Preparation of the macromonomer of formula **M-7** wherein E is hydroxyl, n° is 1, R is $-OC_6H_4-C(CH_3)_2-C_6H_4O-$, $R_1$ is $-CH_3$, $R_4$ is $-C_6H_4-C(CH_3)_2-C_6H_4-$.

100.000 g (0.294 mol) bisphenol-A diglycidyl ether, 134.235 g (0.588 mol) 2,2-bis-(4-hydroxy-phenyl)propane, 83.520 g (0.588 mmol) 2,3-epoxypropylmethacrylate, 2.000 g triethylbenzylammonium chloride, 0.794 g 2,6-di-tert.-butyl-p-cresol (BHT) and 79.439 g triethylenglycol dimethacrylate are homogeneously mixed while heating. The mixture is kept for two hours at 80 °C. The obtained methacrylate terminated macromonomer is soluble in organic solvents such as chloroform, DMF and THF. No absorption of epoxide groups at 915 and 3050 $cm^{-1}$ is observed in the IR-spectrum. Absorption of ester groups is found at 1720 $cm^{-1}$.

**Example 1**

The hydroxyl groups of macromonomer **M-1A** made by following the procedure of reference example 1 are esterified by adding 16.023 g (160.13 mmol) succinic anhydride to 56.900 g of a macromonomer-triethylenglycol dimethacrylate mixture containing 40.000 g (40.03 mmol) macromonomer **M-1A** and 16.9 g of triethylenglycol dimenthacrylate) while stirring for two hours at 90 °C. In the IR-spectrum the esterified macromonomer containing dicarboxylic half ester units shows no absorption of hydroxyl groups at 3400 $cm^{-1}$.

**Example 2**

The hydroxyl groups of macromonomer **M-1B** made by following the procedure of reference example 2 are esterified by adding 197.93 g (1.98 mol) succinic anhydride and 0.56 g triethytamine to 362.71 g macromonomer **M-1B** while stirring for four hours at 90 °C. In the IR-spectrum the esterified macromonomer containing dicarboxylic half ester units shows no absorption of hydroxyl groups at 3400 $cm^{-1}$. The viscosity measured with a Bohlin rheometer is $\eta_{dyn}$ = 245 Pas (25°C).

**Example 3**

The hydroxyl groups of macromonomer **M-1F** made by following the procedure of reference example 3 are esterified by adding 31.58 g (0.32 mol) succinic anhydride. 0.11 g triethylamine and 13.58 g triethyleneglycol dimethacrylate to 107.57 g of a macromonomer-triethylenglycol dimethacrylate mixture (containing 74.40 g, 0.08 mol macromonomer **M-1F**) while stirring for two hours at 90 °C. In the IR-spectrum the esterified macromonomer containing dicarboxylic half ester units shows no absorption of hydroxyl groups at 3400 $cm^{-1}$. $\eta_{dyn}$ = 55.2 Pas (25°C).

**Example 4**

The hydroxyl groups of macromonomer **M-3** wherein each E is hydroxyl made by following the procedure of reference example 4 are esterified by adding to 40.000 g of a macromonomer-triethyleneglycol dimethacrylate mixture (containing 27.844 g, 23.32 mmol macromonomer **M-3** wherein each E is a hydroxy moiety), 9.338 g (93.32 mmol) succinic anhydride and 12.156 g triethylenglycol dimethacrylate while stirring for two hours at 90 °C. The IR-spectrum does not show any absorption of hydroxyl groups at 3400 $cm^{-1}$ of the newly modified macromonomer containing dicarboxylic half ester units.

**Example 5**

The hydroxyl groups of macromonomer **M-5** wherein each E is hydroxyl made by following the procedure of reference example 5 are esterified by adding 23.516 g (235.00 mmol) succinic anhydride to a macromonomer **M-5** wherein each E is hydroxyl for four hours at 90 °C. In the IR-spectrum the esterified macromonomer containing dicarboxylic half ester units show no absorption of hydroxyl groups at 3400 $cm^{-1}$. The macromonomer is characterised by the following analytical data:
Melting point: Fp.= 46.6 °C

| Elemental analysis: ($C_{93}H_{120}N_4O_{24}$) 1678,01 | | |
|---|---|---|
| calc.<br>found | C 66,57H 7,24<br>C 66,60H 6,80 | N 3,34<br>N 2,73 |

**Example 6**

The hydroxyl groups of macromonomer **M-5** wherein each E is hydroxyl made by following the procedure of reference example 5 are esterified by adding 8.239 g (42.88 mmol) trimellitic anhydride, 0.2 g N,N-bis(β-hydroxyethyl)-p-toluidin, 140 ml dioxane and 9.247 g triethylenglycol dimethacrylate to 40.008 g of a macromonomer-triethylenglycol dimethacrylate-mixture (containing 28.000 g, 21.44 mmol macromonomer **M-5** wherein each E is hydroxyl) and kept for eight hours at 90 °C. After evaporation of the dioxane, the macromonomer was washed with petrol ether and dried at 40 °C within six hours. In the IR-spectrum the newly modified macromonomer containing two dicarboxylic half ester units and two hydroxylic groups per average molecule show absorption of hydroxyl groups at 3400 $cm^{-1}$ and of the ester unit at 1720 $cm^{-1}$.

**Example 7**

The hydroxyl groups of macromonomer **M-5** wherein each E is hydroxyl made by following the procedure of reference example 6 are esterified by adding 29.384 g (293.67 mmol) succinic anhydride to a macromonomer and kept for four hours at 90 °C. In the IR-spectrum the esterified macromonomer **M-5** wherein each E is hydrogen (n=0) containing dicarboxylic half ester units shows no absorption of hydroxyl groups at 3400 $cm^{-1}$.

**Example 8**

The hydroxyl groups of macromonomer **M-6** wherein each E is hydroxyl made by following the procedure of reference example 7 are esterified by adding 12.966 g (0.130 mol) succinic anhydride and 0.2 g N,N-bis(β-hydroxyethyl)-p-toluidin to 50.000 g of a macromonomer-triethylenglycol dimethacrylate mixture (containing 35.000 g, 0.032 mol macromonomer **M-6** wherein each E is hydroxyl) while stirring and were kept for eight hours at 50 °C.
In the IR-spectrum the esterified macromonomer containing dicarboxylic half ester units shows no absorption of hydroxyl groups at 3400 $cm^{-1}$.

**Example 9**

The hydroxyl groups of macromonomer **M-7** wherein each E is hydroxyl made by following the procedure of reference example 8 are esterified by adding 12.966 g (0.130 mol) succinic anhydride and 0,2 g N,N-bis(β-hydroxyethyl)-p-toluidin to 50.000 g of a macromonomer-triethylenglycol dimethacrylate mixture (containing 35.000 g, 0.032 mol macromonomer **M-7** wherein each E is hydroxyl) while stirring and kept for two hours at 80 °C. The IR-spectrum does not show any absorption of hydroxyl groups at 3400 $cm^{-1}$ of the esterified macromonomer containing dicarboxylic half ester units.

**Example 10**

The hydroxyl groups of macromonomer **M-6** wherein each E is hydroxyl made by following the procedure of reference example 7 are esterified by adding 29.760 g (0.297 mol) succinic anhydride and 0.2 g N,N-bis(β-hydroxyethyl)-p-toluidin to a macromonomer **M-6** wherein each E is hydroxyl while stirring and were kept for eight hours at 50 °C. In the IR-spectrum the newly modified macromonomer containing dicarboxylic half ester units shows no absorption of hydroxyl groups at 3400 $cm^{-1}$.

**Example 11**

The hydroxyl groups of macromonomer **M-1A** made by following the procedure of reference example 1 are esterified by adding 40.000 g (40.03 mmol) of a macromonomer **M-1A** dissolved in 100 ml THF 16.204 g triethylamine in 50 ml THF. After adding 24.553 g $POCl_3$ (153.33 mmol) drops by drops while stirring at 0° to 5 °C the solution is stirred for further two hours at room temperature. Than the triethylamine hydrochloride is filtered off and the mixture is hydrolysed with 20 ml water. The organic solution is extracted three times with $Na_2CO_3$ solution and is separated from water. From the solution, dried over $MgSO_4$, the solvent is evaporated and the macromonomer is dried.
In the IR-spectrum the esterified macromonomer containing phosphoric ester units shows no absorption of hydroxyl groups at ν=3400 $cm^{-1}$. New absorptions were found at ν= 1007 $cm^{-1}$, ν= 2362 $cm^{-1}$ and as shoulder at ν= 3302 $cm^{-1}$. In the $^1H$ NMR spectrum signals of the olefinic double bonds at $\delta_{(CH2=)}$= 6,0616,12 ppm and at $\delta_{(CH2=)}$= 5,58/5,59 ppm were found. The signals of the methine protons (CH-OP) appears at $\delta_{(CH)}$= 5,22 and 5,88 ppm. Those of unreacted macromonomer ( CH-OH) appears at $\delta_{(CH)}$= 4,34/4,35 ppm.
The HPLC analysis of the modified macromonomer shows the same distribution of oligomers as those of

unreacted **M-1**. Consequently, only the oligomer analogous reaction takes place which does not change the distribution, and no side reaction or crosslinking was observed.

**Example 12**

The hydroxyl groups of macromonomer with **M-3** wherein each E is hydroxyl made by following the procedure of reference example 4 are esterified by adding 60.000 g (50.26 mmol) of a macromonomer **M-3** wherein each E is hydroxyl dissolved in 150 ml THF to 20.346 g triethylamine in 50 ml THF. After adding 30.829 g(201.06 mmol) $POCl_3$ drops by drops while stirring at 0° to 5 °C the solution is stirred for further two hours at room temperature. Than the triethylamine hydrochloride is filtered off and the mixture is hydrolysed with 20 ml water. The organic solution is extracted three times with $Na_2CO_3$ solution and is separated from water. From the solution, dried over $MgSO_4$, the solvent is evaporated and the macromonomer is dried.

In the IR-spectrum the esterified macromonomer containing phosphoric ester units shows no absorption of hydroxyl groups at ν=3400 $cm^{-1}$. New absorptions are found at ν= 1007 $cm^{-1}$, ν= 2362 $cm^{-1}$ and as shoulder at ν= 3302 $cm^{-1}$ and an broad absorption at ν= 2600 to 2800 $cm^{-1}$ of the ammonium salt.

**Example 13**

The hydroxyl groups of macromonomer **M-6** wherein each E is hydroxyl made by following the procedure of reference example 7 are esterified by adding 40.000 g (37.83 mmol) of a macromonomer **M-6** wherein each E is hydroxyl dissolved in 100 ml THF to 15.312 g triethyiamine in 50 ml THF. After adding 23.200 g (151.31 mmol) $POCl_3$ drops by drops while stirring at 0° to 5 °C the solution is stirred for further two hours at room temperature. Than the triethylamine hydrochloride is filtered off and the mixture is hydrolysed with 20 ml water. The organic solution is extracted three times with $Na_2CO_3$ solution and is separated from water. From the solution, dried over $MgSO_4$, the solvent is evaporated and the macromonomer is dried.

In the IR-spectrum the esterified macromonomer containing phosphoric ester units shows no absorption of hydroxyl groups at ν=3400 $cm^{-1}$. New absorptions are found at ν= 1007 $cm^{-1}$, ν= 2362 $cm^{-1}$ and as shoulder at ν= 3302 $cm^{-1}$.

**Example 14**

1) 75 % of hydroxyl groups of the macromonomer **M-1A** made by following procedure of reference example 1 are esterified with succinic acid anhydride by adding 148.387 g (0.116 mol) of a macromonomer **M-1A** to 34.890 g (0.349 mol) succinic anhydride and 0.183 g triethylamine and reacted for two hours at 80°C while stirring. The macromonomer is dissolved in 250 ml THF and stirred for a further hour. The esterified macromonomer **M-1A** containing (n+2)-carboxylic half ester groups show in the IR-spectrum an absorption of $\nu_{CO}$ =1720 $cm^{-1}$.

2) Esterification of the residual unreacted hydroxyl groups of the macromonomer with $POCl_3$.

To 183.460 g (0.141 mol) of the obtained macromonomer **M-1A** dissolved in 250ml THF were added 14.287 g triethylamine in 50 ml THF. After adding 21.659 g (0.141 mol) $POCl_3$ drops by drops while stirring at 0° to 5 °C the solution is stirred for further two hours at room temperature. Than the triethylamine hydrochloride is filtered off and the mixture is hydrolysed with 50 ml water. The organic solution is extracted three times with $Na_2CO_3$ solution and is separated from water. From the solution, dried over $MgSO_4$, the solvent is evaporated and the macromonomer is dried.

In the IR-spectrum the esterified macromonomer containing phosphoric ester units shows no absorption of hydroxyl groups at ν=3400 $cm^{-1}$. New absorptions are found at ν= 1007 $cm^{-1}$, ν= 2362 $cm^{-1}$ and as shoulder at ν= 3302 $cm^{-1}$. In the $^1H$ NMR spectrum signals of the olefinic double bonds at $\delta_{(CH2=)}$= 6,06/6,12 ppm and at $\delta_{(CH2=)}$= 5,58/5,59 ppm were found. The signals of the methine protons (CH-O-P) appear at $\delta_{(CH)}$= 5,22 and 5,88 ppm. Those containing succinic half ester units appear at $\delta_{(CH)}$= 5,38 ppm. The esterified macromonomer **M-1** containing (n+2)-carboxytic half ester groups and n-phosphoric acid groups is described by the following formula (n°=1, R=$-OC_6H_4-C(CH_3)_2-C_6H_4O-$,$R_2$= $-(CH_2)_4-$, $R_5$=$-CH_2CH_2-$):

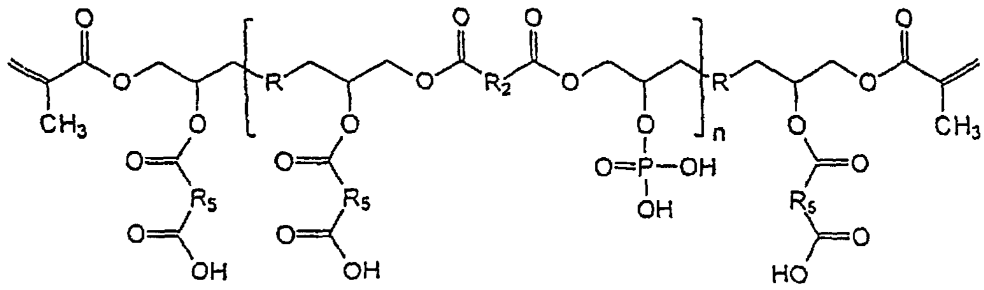

**Example 15**

100.00 g (161.29 mmol) of a monophosphate ester of pentaerythrit pentmethacrylate and 27.29 g (161.29 mmol) dimethylaminoethyl methacylate are dissolved in 84.86 g triethyleneglycol dimethacrylate and reacted for two hours at 50°C. In the IR spectrum at 2600 to 2850 cm-1 an absorption of the ammonium salt is found.
At 3400 $cm^{-1}$ no absorption of OH-groups is observed. The pH of the salt is 3,9.

**Application Example 1 (Dental adhesive)**

1.242 g of the esterified macromonomer **M-5** wherein each E is succinic acid half ester made by following the procedure of example 5, 0.411 g triethyleneglycol dimethacrylate, 0.008 g N,N-bis(β-hydroxyethyl)-p-toluidine and 0.006 g camphorquinone were homogeneously mixed. This mixture was applied in a ring (2mm high, 5 mm i.d.) on the surface of teeth and exposed with visible light (irradia-tion lamp Prismetics Lite De Trey Dentsply) for 40 seconds. Immediately after fixa-tion, the teeth are transferred for 24 hours to a chamber at 37 ± 2 °C and 100 % relative humidity. The adhesion measured with a Zwick-apparatus is 3,74 ± 1,29 MPa.

**Comparative Example 1**

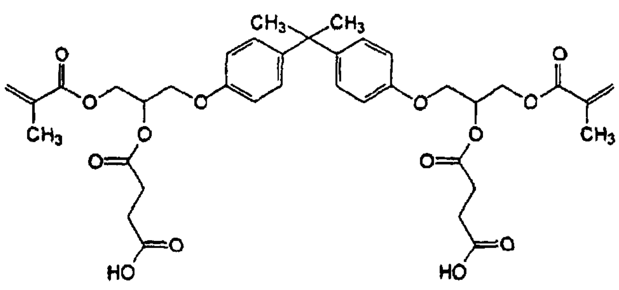

2.420 g of 2,2-Bis-[p-(2-hydroxy-3-methacryloyloxypropoxy)-phenyl]-propan (Bis-GMA) which is modified with succinic anhydride at the hydroxyl groups, 0.821 g triethylenglycol dimethacrylate, 0,016 g N,N-bis(β-hydroxyethyl)-p-toluidine and 0.012 g camphorquinone were homogeneously mixed. This mixture is applied in a ring (2mm high, 5 mm i.d.) on the surface of teeth and exposed with visible light (irradia-tion lamp Prismetics Lite De Trey Dentsply) for 40 seconds. Immediately after fixa-tion, the teeth are transferred for 24 hours to a chamber at 37 ± 2 °C and 100 % relative humidity. The adhesion is 0,45 ± ,20 Mpa, when measured with a Zwick-apparatus model number 1455, manufactured by Zwick GmbH & Co.

***Application Example 2 (Dental adhesive)***

1.276 g of the esterified macromonomer **M-5** wherein each E is succinic acid half ester made by following the procedure of example 5, 2.126 g triethylenglycol dimethacry-late, 6.5 g Strontium-alumo-silicate glass, 0.036 g camphorquinone and 0.045 g N,N-bis(β-hydroxyethyl)-p-toluidine are homogeneously mixed and polymerized photochemical. The product has the following properties; adhesin to dentine of 3.7 ± 1.1 MPa, compressive strength 177 ± 3.5 MPa, Elastic Modulus of 2383 ± 71 MPa.

***Application Example 3 (Dental adhesive)***

1.755 g of macromonomer **M-5** wherein each E is succinic acid half ester of example 5, 0.752 g methylmethacrylate, 4.652 g Strontium-alumo-silicate glass, 0.010 g camphorquinone and 0.012 g N,N-bis(β-hydroxyethyl)-p-toluidine are homogeneously mixed and polymerized photochemically. The product obtained has the following properties: adhesion to dentine: 3.9 ± 1.2 MPa, compressive strength 134 ± 9.7 MPa, Elastic Modulus 2528 ± 158 MPa.

***Application Example 4 (Dental* adhesive)**

Paste A:

3.0404 g of macromonomer **M-5** wherein each E is succinic acid half ester of example 5, 2.2512 g triethylenglycol dimethacrylate, 6.0 g $CaWO_4/ZrO_2$ (80/20) and 0.3135g Strontium-alumo-silicate glass containing 10% lithium-sulfinate are homogeneously mixed.

Paste B):

3.0404 g of macromonomer **M-5** wherein each E is succinic acid half ester of example 5, 2.2512 g triethylenglycol dimethacrylate, 6.0 g $CaWO_4/ZrO_2$ (80/20), 0.0057 g octophen and 0.0668 g Strontium-alumo-silicate glass containing 1% Cu-(I)-thiourea complex are homogeneously mixed.

Immediately before use paste A and paste B were mixed in the wt.-ratio 1:1 homogeneously. The gel time at 23°C is estimated to be 32 min. and the gel time at 23°C is 7 minutes. The radio-opacity (RO) of the obtained material is 6.5 mm/mm Al.

***Application Example 5 (Dental adhesive)***

Paste A:

8.001 g of macromonomer **M-1A** of example 1, 5.334 g triethylenglycol dimethacrylate, 14.467 g $CaWO_4/ZrO_2$ (80/20), 0.014 g 2.6-di-tert.-butyl-p-cresol and 0.533g Strontium-alumo-silicate glass containing 10% lithium-sulfinate are homogeneously mixed.

Paste B:

8,001 g of macromonomer **M-1A** of example 1, 5.334 g triethylenglycol dimethacrylate, 14.467 g $CaWO_4/ZrO_2$ (80/20), 0.014 g 2.6 -di-tert.-butyl-p-cresol, 0.065 g octophen and 0.0533 g Strontium-alumo-silicate glass containing 1% Cu-(I)-thiourea complex are homogeneously mixed.

Immediately before use paste A and paste B were mixed in the wt.-ratio 1:1 homogeneously. The gel time at 23°C is about 96 minutes, and the gel time at 32°C is 19 min. The radioopacity of the obtained material is about about 6.7 mm/mm Al.

***Application Example 6 (Dental adhesive)***

Powder:

15,000 g silylated Strontium-alumo-silicate glass and 2,000 g silylated Strontium-alumo-silicate glass containing 10% dibenzoylperoxide were mixed homogeneously.

Liquid:

14.000 g of a macromonomer **M-5** wherein each E is succinic acid half ester of example 5, 6.000 g tetrahydrofurfuryl-methacrylate, 0.405 g N,N-bis(β-hydroxyethyl)-p-toluidine, 0.0130 g 2,6-di-tert.-butyl-p-cresol are mixed homogeneously.

Immediately before use powder and liquid were mixed in the wt.-ratio 1,73:1,00 homogeneously. The working time is 3.50 minutes and the setting time is 3.25 minutes. The adhesion to dentine is measured to be 2,2 ± 0,7 MPa. The composite shows the following mechanical properties: compressive strength: 152 ± 15 MPa, and elastic modulus of 1788 ± 81 MPa.

***Application Example 7 (Dental adhesive)***

Paste A:

3.000 g of an ammonium salt of dipenta erthrytrol pentamethacrylate monophosphate and 2-(dimetyl)aminoethyl methacrylate (**AP-1**), 2.000 g macromonomer **M-1A** of example 1, 5.000 g triethylenglycol dimethacrylate, 15.000 g Strontium-alumo-silicate glass, 0.005 g 2,6-di-tert.-butyl-p-cresol and 0.200 g cumenhydroperoxide are mixed homogeneously.

Paste B:

3.000 g of **AP-1,** 2,000 g macromonomer **M-1A** of exapmple 1, 5.000 g triethylenglycol dimethacrylate, 15.000 g Strontium-alumo-silicate glass, 0.005 g 2,6-di-tert.-butyl-p-cresol, 0.4081 g of a 0,1 % solution of cupric acetylacetonate in 2-hydroxy propylmethacrylate and 0.041 g ascorbic acid palmitate are mixed homogeneously.
The following values are measured: gel time (gt) at 23°C is 3:55 minutes, gel time is 37°C is 2.10 minutes, adhesion to dentine 5.12 MPa, shrinkage (reduction in volume) is 4.33 %.

***Application Example 8 (Dental adhesive)***

Paste A:

3.000 g of **AP-1,** 2,000 g of macromonomer **M-1A** of exapmple 1, 5.000 g triethylenglycol dimethacrylate, 15.000 g Strontium-alumo-silicate glass, 0.005 g 2,6-di-tert.-butyl-p-cresol and 0.200 g tert.-butyl peroxy benzoate are mixed homogeneously.

Paste B:

3.000 g of **AP-1**, 2.000 g macromonomer **M-1A** of exapmple 1, 5.000 g triethylenglycol dimethacrylate, 15.000 g Strontium-alumo-silicate glass, 0.005 g 2,6-di-tert.-butyl-p-cresol, 0.6186 g of a 0,1 % solution of cupric acetyacetonate in 2-hydroxypropylmethacrylate and 0.051 g ascorbic acid palmitate are mixed homogeneously.
The following values were measured: gel time (gt) at 23°C is 6.10 minutes, (gt) at 37°C is 3.20 minutes, adhesion to dentine 4.02 MPa, shrinkage (or reduction in volume) is 4.33 %.

***Application Example 9 (Dental adhesive)***

Powder:

41.842 g silylated Strontium-alumo-silicate glass and 0.423 g dibenzoyl peroxide are mixed homogeneously.

Liquid:

18.000 g of **AP-1,** 12.000 g triethylenglycoldimethacrylate, 0.180 g N,N-dimethyl-3,5-dimethyl aniline and 0,009 g 2,6-di-tent.-butyl-p-cresol were mixed homogeneously. Immediately before use powder and liquid were mixed in the weight ratio 1.40:1,00 homogeneously. The working time is 1:30 minutes and the setting time is 2:30 minutes.
The following properties are measured:

| | |
|---|---|
| adhesion to dentine | 7.68 ± 1,5 MPa |
| compressive strength | 261 ± 14 MPa |
| Elastic modules | 2917 ± 76 MPa |
| shrinkage (percent reduction in volume) | 2.30 % |
| expansion (expansion in length) | 1.17 % (after storage for 14 weeks in water at 37°C) |
| fluoride release | 5.33 µg/cm$^2$ (after storage for 9 weeks in water at 37°C). |

***Application Example 10 (Dental adhesive)***

Powder:

47.0 g silylated Strontium-alumo-silicate glass, 07544 g dibenzoyl peroxide and 2.52 g $SrF_2$ are mixed homogeneously.

Liquid:

28.570 g of an ammonium salt of dipentaerthrytrolpentamethacrylate monophosphate and 2-(dimetyl)aminoethyl methacrylate **(AP-1)** containing 8.570 g triethylenglycol dimethacrylate, 7.140 g macromonomer **M-1A** of example 1 containing 2.140 g triethylenglycol dimethacrylate, 13.990 g triethylenglycol dimethacrylate 0.250 g N,N-bis (β-hydroxyethyl)-p-toluidine and 0.05 g 2,6-di-tert.-butyl-p-cresol are mixed homogeneously.

Immediately before use powder and liquid are mixed in the weight ratio 1.40:1.00 homogeneously. The working time is 5:50 minutes and the setting time is 4:15 minutes.

The following properties are measured:

| | |
|---|---|
| adhesion to dentine | 7,7 ± 0,8 MPa |
| compressive strength | 295 ± 9 MPa (ISO 9917, EN 29917) |
| flexural strength | 77,1 ± 7,1 MPa (ISO 4049, EN 24049) |
| Elastic modulus | 4482 ± 147 MPa (ISO 4049, EN 24049) |
| Opacity | 90.6 % (ISO 9912, EN 29912) |
| shrinkage ΔV | 5.8 ± 0.5 % |
| expansion ΔL | 1.52 % (after storage for 28 weeks in water at 37°C) |
| fluoride release | 64.01 μg/cm$^2$ (after storage for 27 weeks in water at 37°C). |

***Application Example 11* (Dental *adhesive)***

Powder:

46.2480 g silylated Strontium-alumo-silicate glass, 0.6937 g dibenzoyl peroxide and 2.3124 g $SrF_2$ are mixed homogeneously.

Liquid:

20.0000 g of **AP-1** containing 14.0000 g triethylenglycol dimethacrylate, 5.0000 g macromonomer **M-1A** of example 1 containing 1.5000 g triethylenglycol dimethacrylate, 1.323 g demineralised water, 4.0000 g UDMA, 4.7000 g triethylenglycol dimethacrylate, 0.1485 g N,N-bis(β-hydroxyethyl)-p-toluidine and 0.0098 g 2,6-di-tert.-butyl-p-cresol are mixed homogeneously. The viscosity measured with a Bohlin rheometer is $\eta_{dyn}$ = 1.086 ± 0.005 Pas (23°C).

Immediately before use powder and liquid are mixed in the weight ratio 1.40:1.00 homogeneously. The working time is 4:00 minutes and the setting time is 4:00 minutes.

The following properties were measured:

| | |
|---|---|
| adhesion to dentine | 5.86 ± 1.53 MPa |
| compressive strength | 301 ± 11 MPa |
| flexural strength | 74.8 ± 4.8 MPa |
| Elastic modulus | 5320 ± 271 MPa |
| expansion ΔL | 1.10 % (after storage for 5 weeks in water at 37°C) |
| fluoride release | 114.05 pg/cm$^2$ (after storage for 28 weeks in water at 37°C). |

***Application Example 12 (Dental sealant)***

20.00 g macromonomer **M-1A** of example 1, 19.63 g triethylenglycol dimethacrylate, 0.18 g N,N-bis(β-hydroxyethyl)-p-toluidine, 0.12 g camphorquinone, 0.07 g 2,6-di-tert.-butyl-p-cresol and 60.00 g Strontium-alumo-silicate glass are mixed homogeneously. Results are given in table 1.

***Application Example 13 (Dental sealant)***

20.00 g macromonomer **M-1A** of example 14, 19.63 g triethylenglycol dimethacrylate, 0.18 g N,N-bis(β-hydroxyethyl)-p-toluidine, 0.12 g camphoro quinone, 0.07 g 2,6-di-tert.-butyl-p-cresol and 60.00 g Strontium-alumo-silicate glass are mixed homogeneously. Results are given in table 1.

**Comparative Example 2**

20.00 g of 2,2-Bis-[p-(2-hydroxy-3-methacryloyloxypropoxy)-phenyl]-propan (Bis-GMA) which is modified with succinic anhydride at the hydroxyl groups, 19.63 g triethylenglycol dimethacrylate, 0.18 g N,N-bis(β-hydroxyethyl)-p-toluidine, 0.12 g camphorquinone, 0.07 g 2,6-di-tent.-butyl-p-cresol and 60.00 g Strontium-alumo-silicate glass are mixed homogeneously. Results are given in table 1.

Table 1

| | Macromonomer **M-1A** according example 12 | Macromonomer **M-1A** according example 13 | Bis-GMA according comparative example 2 |
|---|---|---|---|
| Adhesion to dentin MPa | 2.35 | 2.42 | 1.04 |
| Standard deviation MPa | ± 0.79 | ± 1.06 | ± 0.34 |
| Molecular weight of modified Macromonomer | 1399.4 | 1379.3 | 712.3 |
| Molecular weight per ester unit | 349.9 | 344.8 | 356.2 |

***Application Example 14 (Dental sealant)***

10.00 g of **AP-1** containing 3.00 g triethylenglycol dimethacrylate, 2.50 g macromonomer **M-1A** of example 1 containing 0.75 g triethylenglycol dimethacrylate, 1.25 g triethylenglycol dimethacrylate, 0.0875 g N,N-bis(β-hydroxyethyl)-p-toluidine, 0.0875 g camphor quinone, 11.49 g Strontium-alumo-silicate glass, 0.30 g Aerosil and 0.0088 g 2,6-di-tert.-butyl-p-cresol are mixed homogeneously. The viscosity measured with a Bohlin rheometer is $\eta_{dyn}$ = 1.086 ± 0.005 Pas (23°C).

***Application Example 15 (Dental/medical composite)***

2.000g macromonomer **M-6** of example 8 containing 0.400 g triethylenglycol dimethacrylate, 5.273 g Strontium-alumo-silicate glass, 0.010 g champhorquinon and 0.012 g N,N-bis(β-hydroxyethyl)-p-toluidin are homogeneously mixed and polymerized photochemical. The composite shows the following mechanical properties:

| | |
|---|---|
| flexural strength | 76.6 ± 4.5 MPa |
| flexural modules | 5074.0 ± 321 MPa |
| compressive strength | 215.0 ± 6.0 MPa |
| Elastic modules | 3180.0 ± 88 Mpa |

## Claims

1. An esterified macromonomer obtainable by esterification of at least a portion of -OH groups of an OH- group containing macromonomer having at least one terminal double bond with at least one derivative of an inorganic or organic acid whereby pendant groups are introduced exhibiting at least one acid moiety selected from the group consisting of-COOH, $-PO_3H_2$, $-SO_3H$, - $BO_2H$ or salts thereof, whereby the esterified macromonomer is within the scope of the general formula:

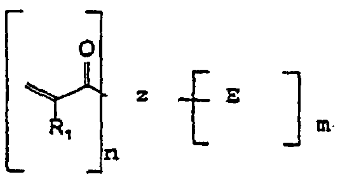

wherein Z is an organic moiety,

| | |
|---|---|
| $R_1$ | is hydrogen or a substituted or unsubstituted alkyl having from 1 to 12 carbon atoms, oxyalkyl having from 1 to 12 carbon atoms, alkenyl having from 2 to 12 carbon atoms, cycloalkyl having from 5 to 12 carbon atoms, aryl having from 6 to 12 carbon atoms or aralkyl having from 7 to 12 carbon atoms, |
| each E | independently is a hydroxyl group or an organic or inorganic ester moiety and at least one E is an organic or inorganic ester moiety, |
| n and m | each independently is an integer from 2 to 12. |

**2.** The esterified macromonomer of claim 1 wherein said esterified macromonomer is within the scope at least one of formulas **M-1** to **M-12:**

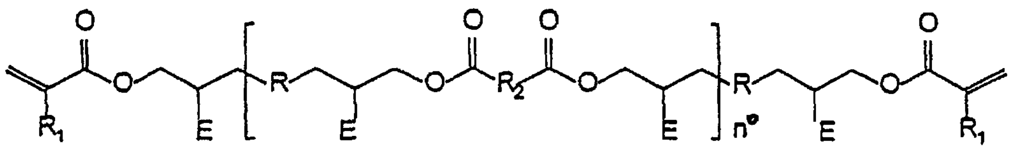

M-1

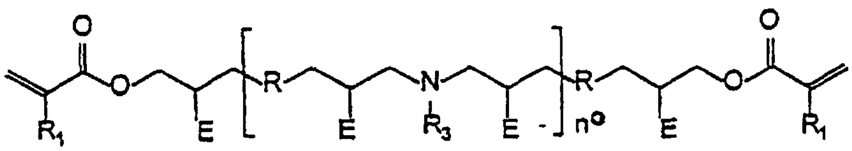

M-2

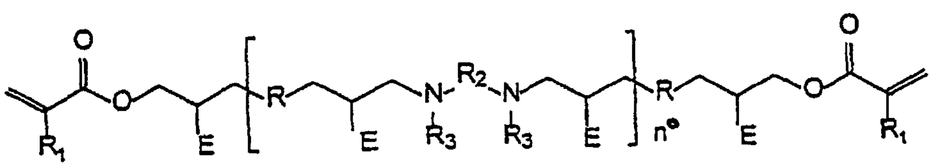

M-3

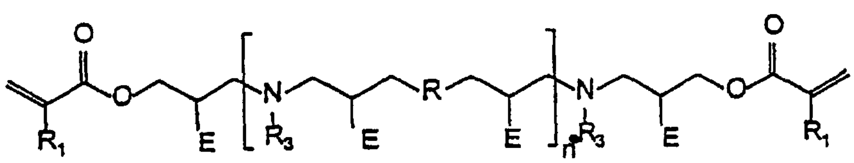

M-4

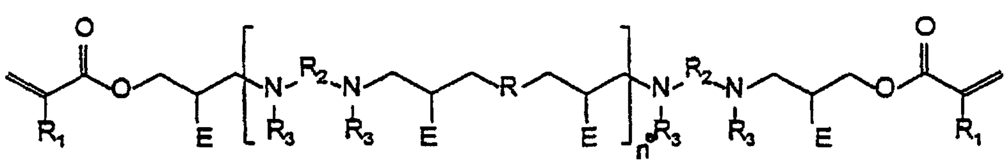

M-5

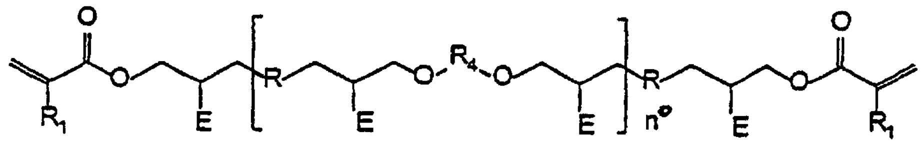

M-6

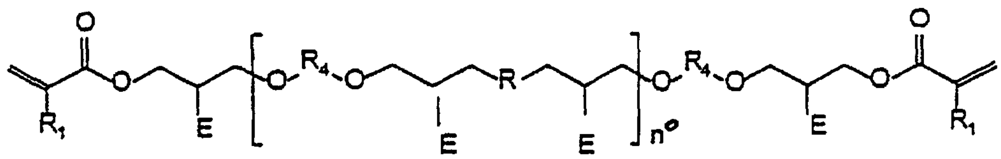

M-7

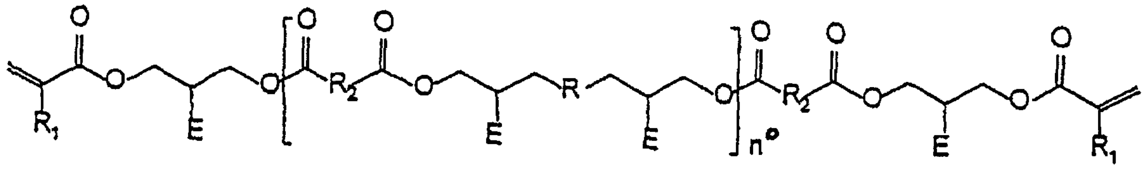

M-8

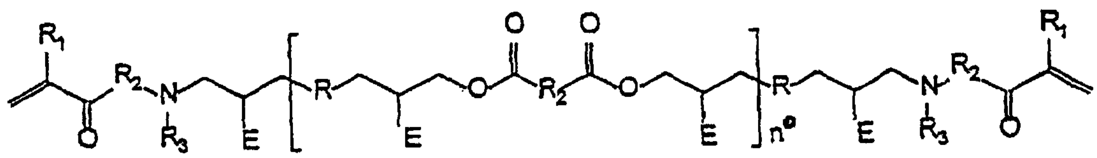

M-9

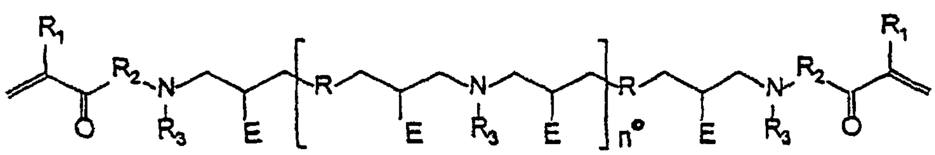

M-10

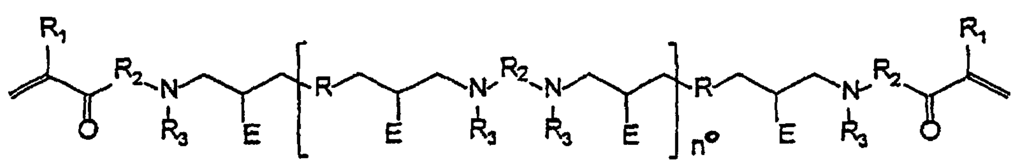

M-11

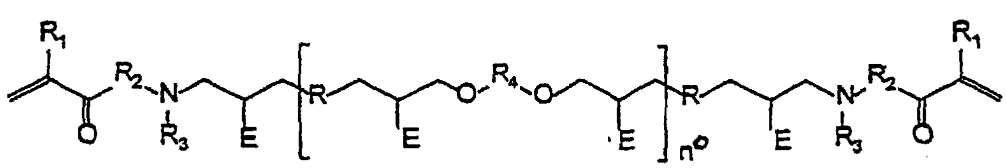

M-12

wherein E and $R_1$ are as defined as in claim 1,

R is a diether containing moiety, or diester containing moiety or tertiary amine containing moiety,

$R_2$ is a difunctional substituted or unsubstituted alkyl group having from 1 to 12 carbon atoms, alkenyl group having from 2 to 12 carbon atoms, cycloalkyl having from 5 to 12 carbon atoms, aryl having from 6 to 12

carbon atoms or aralkyl having from 7 to 12 carbon atoms,

$R_3$ is hydrogen or a substituted or unsubstituted alkyl group having from 1 to 12 carbon atoms, alkenyl group having from 2 to 12 carbon atoms, cycloalkyl having from 5 to 12 carbon atoms, aryl having from 6 to 12 carbon atoms or aralkyl having from 7 to 12 carbon atoms,

$R_4$ is a substituted or unsubstituted aryl having from 6 to 12 carbon, and

n° is an integer of at least 1.

**3.** The esterified macromonomer of claim 2 wherein R is within the scope of the general formula:

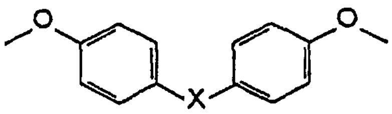
,
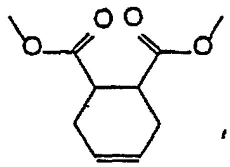
,
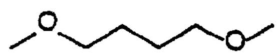
or
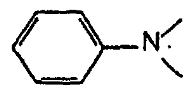

wherein X is >$C(CH_3)_2$, $-CH_2-$, -O- -S-, -CO-, or $-SO_2-$

**4.** The esterified macromonomer of claim 2 wherein R is

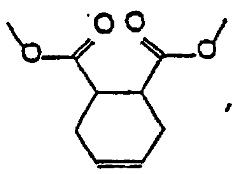
,
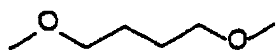
or
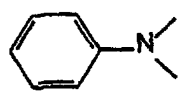

**5.** The esterified macromonomer of claim 2 wherein $R_4$ is within the scope of at least one of the general formulas:

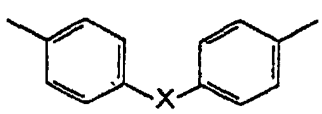
or
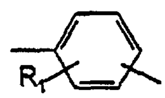

wherein X is >$C(CH_3)_2$, $-CH_2-$, -0-, -S-, -CO-, or $-SO_2-$.

**6.** The esterified macromonomer of claim 2 wherein at least one E comprises a carboxyl group.

**7.** The esterified macromonomer of claim 1 wherein E is derived from succinic acid anhydride, maleic acid anhydride, dichloro maleic acid anhydride, dimethyl maleic acid anhydride, malonic acid anhydride, aconit acid anhydride, adipic acid anhydride, 3,3-tetramethylen glutaric acid anhydride, cyclohexen-1,2 acid anhydride, nadinic acid anhydride, phthalic acid anhydride, trimellitic acid anhydride, 2-sulfo-benzoic acid anhydride, 2-sulfo succinic acid anhydride, phthalic acid anhydride p-(O-phosphate), phthaloylchloride, succinic acid dimethyl ester, phosphorous penta chloride, phosphorous trichloride, phosphorous oxychloride, sulfuryl chloride, thionyl chloride, phosphor thionyl chloride, boric acid anhydride and boron trichloride.

**8.** The esterified macromonomer of claim 1 wherein at least one E is a salt selected from the group consisting of ammonium, sulfonium, sodium, potassium, strontium, calcium and magnesium salts.

**9.** The esterified macromonomer of anyone of claims 1 to 8, wherein said esterification is carried out in a solvent selected from the group consisting of THF, triethylenglycol bismethacrylate, diethylenglycol bismethacrylate, dioxolan bismethacrylate, vinyl-, vinylen- or vinyliden, acrylate- or methacrylate substituted sprio-orthoester, spiroorthocarbonate or bicycloorthoester and 2,2-Bls[p-(acryloxyethoxy)phenyl]propane.

**10.** The esterified macromonomer of anyone of claims 1 to 8, wherein said esterification is carried out in the presence

of a tertiary amine.

**11.** An esterified macromonomer according to claim 1, wherein the number of said acid moieties is chosen such that a polymer obtained by polymerizing said monomer has an adhesive strength to dentine of at least 2 MPa.

**12.** A dental/medical composition comprising the macromonomer of claim 2, a filler, a polymerizable monomer having at least one phosphorous ester group, a polymerization initiator and a stabilizer.

**13.** The composition according to claim 12 further comprising a polymerizable monomer selected from the group consisting of mono- and polyfunctional (meth)acrylate, a urethane di- and poly(meth)acrylate, a vinyl-, vinylen- or vinyliden-, acrylate- or methacrylate substituted spiro-orthoester, a spiroorthocarbonate or a bicyloorthoester, and said monomer comprises from about 5 to about 80 percent by weight.

**14.** The composition according to claim 12 wherein said polymerization initiator is a thermal initiator, a redox-initiator or a photoinitiator.

**15.** The composition according to claim 12 wherein said filler comprises an inorganic filler and/or an organic filler.

**16.** The composition according to claim 15 wherein said filler is a fluoride releasing inorganic filler.

**17.** The composition formed by polymerizing the composition of claim 12 to form a polymeric product having an adhesion to dentine of at least 2 MPa, a fluoride release of at least 1 $\mu g\ F^-$ per week and per $cm^2$ of the exposed surface of the composition, an opacity of at least $C_{0,7}$= 40 % and a compressive strength of at least 200 MPa.

**18.** The composition of claim 12 comprising from about 5 to about 20 percent by weight of said esterified macromonomer, from about 10 to about 25 percent by weight of a di- or poly(meth)acrylate monomer having at least one phosphorous acid ester group, from about 20 to about 35 percent by weight of a polymerizable monomer, from about 50 to about 65 percent by weight of a filler and polymerization initiator and stabilizers.

**19.** The composition of claim 12 comprising from about 3 to about 15 percent by weight of said esterified macromonomer, from about 5 to about 25 percent by weight of di- or poly(meth)acrylate monomer having at least one phosphorous acid ester group, from about 7 to about 40 percent by weight of a polymerizable monomer, from about 50 to about 85 percent by weight of a filler and polymerization initiator and stabilizers.

**20.** The composition of claim 12 comprising from about 5 to about 25 percent by weight of said esterified macromonomer, from about 10 to about 30 percent by weight of di- or poly(meth)acrylate monomer having at least one phosphorous acid ester group, from about 20 to about 40 percent by weight of a polymerizable monomer, from about 10 to about 50 percent by weight of a filler and polymerization initiator and stabilizers.

**21.** The composition of claim 12 comprising from about 5 to about 25 percent by weight of said esterified macromonomer, from about 5 to about 30 percent by weight of di- or poly(meth)acrylate monomer having at lest one phosphorous acid ester group, from about 40 percent by weight of a polymerizable monomer, from about 30 to about 90 percent by weight of a diluent and initiator an stabilizers.

**22.** The composition of claim 12 comprising about 1 to about 25 percent by weight of said esterified macromonomer, di- or poly(meth)acrylate monomer having at least one phosphous acid ester group and a polymerizable monomer and polymerization initiator from about 75 to about 99 percent by weight of an organic solvent and polymerization co-initiator.

## Revendications

**1.** Macromonomère estérifié pouvant être obtenu par estérification d'au moins une partie des groupes -OH d'un macromonomère contenant des groupes OH ayant au moins une double liaison terminale avec au moins un dérivé d'un acide inorganique ou organique, ce qui provoque l'introduction de groupes appendus présentant au moins un groupement acide choisi dans le groupe consistant en -COOH, $-PO_3H_2$, $-SO_3H$, $-BO_2H$ ou leurs sels, ledit macromonomère estérifié répondant à la formule générale :

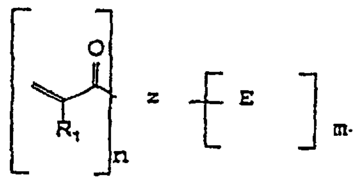

dans laquelle Z représente un groupement organique,

| | |
|---|---|
| $R_1$ | représente l'hydrogène ou un groupe, substitué ou non substitué, alkyle ayant 1 à 12 atomes de carbone, oxyalkyle ayant 1 à 12 atomes de carbone, alcényle ayant 2 à 12 atomes de carbone, cycloalkyle ayant 5 à 12 atomes de carbone, aryle ayant 6 à 12 atomes de carbone ou aralkyle ayant 7 à 12 atomes de carbone, |
| chaque symbole E | représente, indépendamment, un groupe hydroxyle ou un groupement ester organique ou inorganique et au moins un groupement E est un groupement ester organique ou inorganique, |
| n et m | représentent, indépendamment, un nombre entier de 2 à 12. |

**2.** Macromonomère estérifié suivant la revendication 1, qui répond à au moins une des formules M-1 à M-12 :

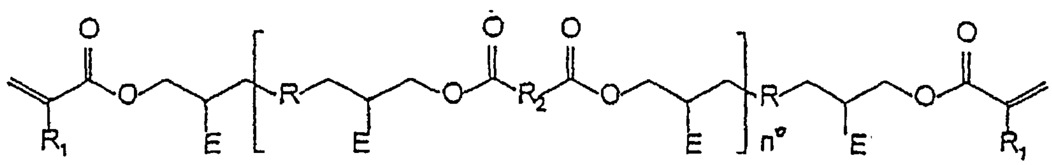

M-1

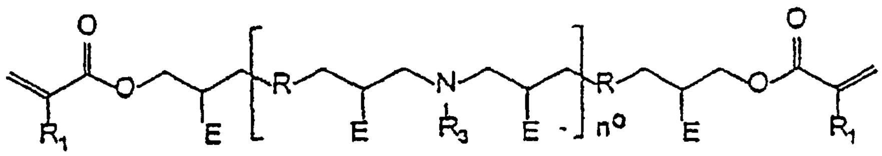

M-2

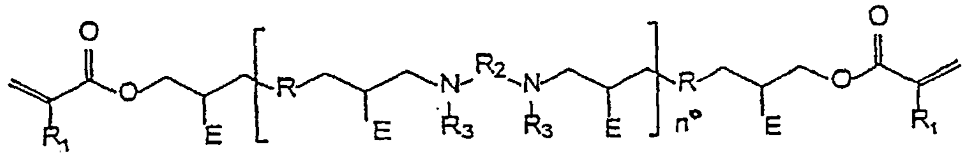

M-3

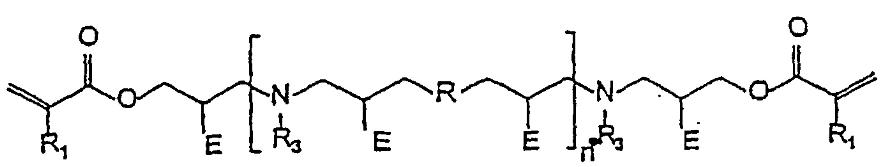

M-4

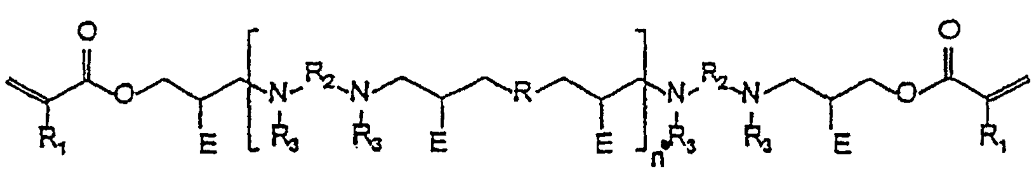

M-5

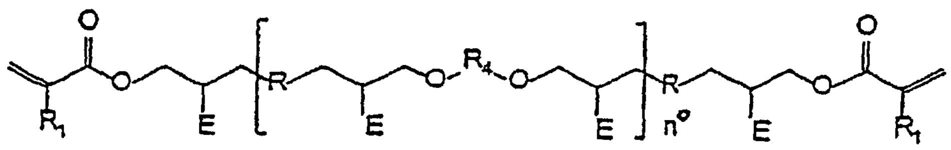

M-6

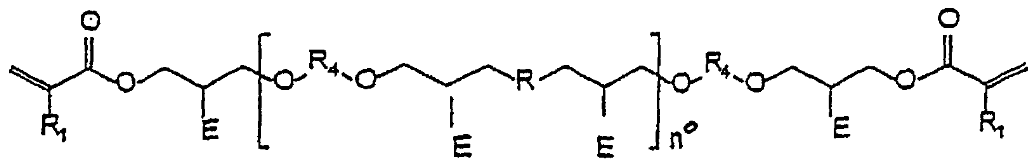

M-7

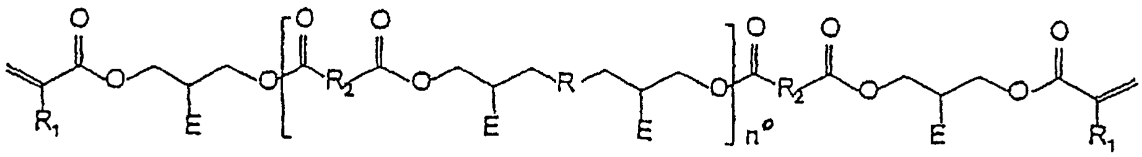

M-8

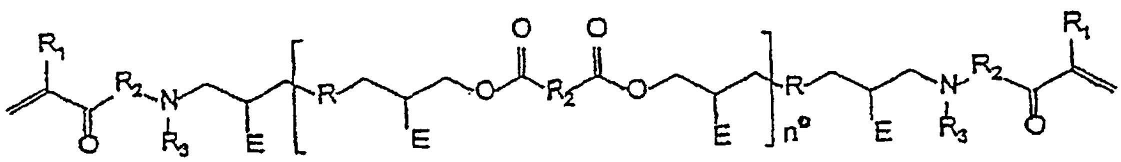

M-9

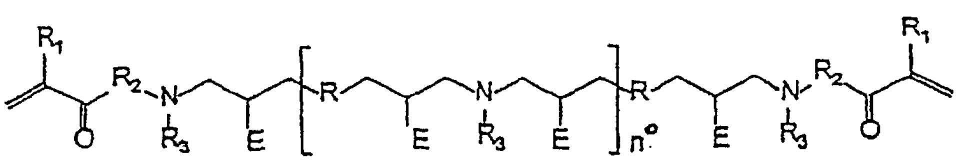

M-10

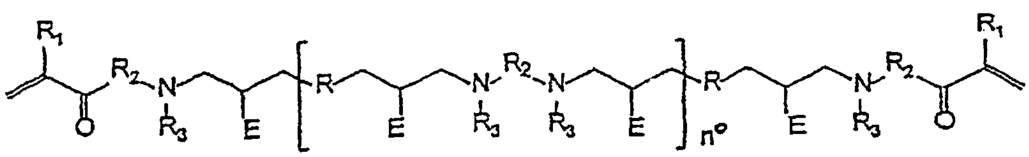

M-11

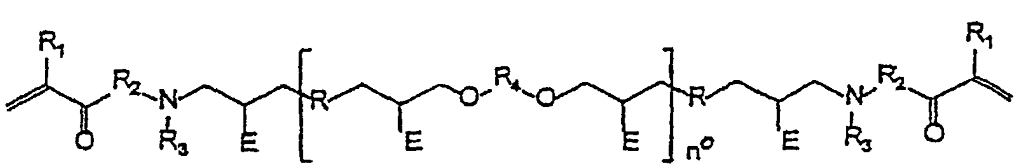

M-12

dans lesquelles E et $R_1$ répondent aux définitions suivant la revendication 1,

- R représente un groupement à fonction diéther, ou un groupement à fonction diester ou un groupement à fonction aminé tertiaire,
- $R_2$ représente un groupe alkyle difonctionnel, substitué ou non substitué, ayant 1 à 12 atomes de carbone, un groupe alcényle ayant 2 à 12 atomes de carbone, cycloalkyle ayant 5 à 12 atomes de carbone, aryle ayant 6 à 12 atomes de carbone ou aralkyle ayant 7 à 12 atomes de carbone,

$R_3$ représente l'hydrogène ou un groupe, substitué ou non substitué, alkyle ayant 1 à 12 atomes de carbone, alcényle ayant 2 à 12 atomes de carbone, cycloalkyle ayant 5 à 12 atomes de carbone, aryle ayant 6 à 12 atomes de carbone ou aralkyle ayant 7 à 12 atomes de carbone,

$R_4$ représente un groupe aryle, substitué ou non substitué, ayant 6 à 12 atomes de carbone, et

n° représente un nombre entier égal à au moins 1.

**3.** Macromonomère estérifié suivant la revendication 2, dans lequel R répond à la formule générale :

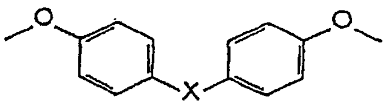
,
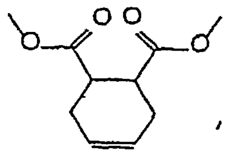
,
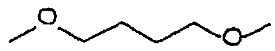
ou
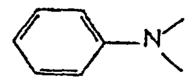

dans laquelle X représente un groupe >$C(CH_3)_2$, $-CH_2-$, -O-, -S-, -CO- ou $-SO_2-$.

**4.** Macromonomère estérifié suivant la revendication 2, dans lequel R représente un groupe

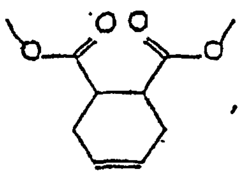
,
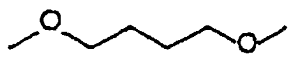
ou
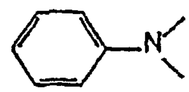

**5.** Macromonomère estérifié suivant la revendication 2, dans lequel $R_4$ répond à au moins une des formules générales :

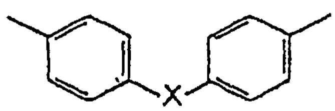
ou
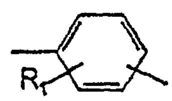

dans lesquelles X représente un groupe >$C(CH_3)_2$, $-CH_2-$, -O-, -S-, -CO- ou $-SO_2-$.

**6.** Macromonomère estérifié suivant la revendication 2, dans lequel au moins un groupement E comprend un groupe carboxyle.

**7.** Macromonomère estérifié suivant la revendication 1, dans lequel E est dérivé de l'anhydride d'acide succinique, de l'anhydride d'acide maléique, de l'anhydride d'acide dichloromaléique, de l'anhydride d'acide diméthylmaléiqué, de l'anhydride d'acide malonique, de l'anhydride d'acide aconitique, de l'anhydride d'acide adipique, de l'anhydride d'acide 3,3-tétraméthylène-glutarique, de l'anhydride d'acide cyclohexène-1,2-carboxylique, de l'anhydride d'acide nadinique, de l'anhydride d'acide phtalique, de l'anhydride d'acide trimellitique, de l'anhydride d'acide 2-sulfobenzoïque, de l'anhydride d'acide 2-sulfosuccinique, du p-(O-phosphate) d'anhydride d'acide phtalique, du chlorure de phtaloyle, de l'ester diméthylique d'acide succinique, du pentachlorure de phosphore, du trichlorure de phosphore, de l'oxychlorure de phosphore, du chlorure de sulfuryle, du chlorure de thionyle, du chlorure de phosphorothionyle, de l'anhydride d'acide borique ou du trichlorure de bore.

**8.** Macromonomère estérifié suivant la revendication 1, dans lequel au moins un groupement E est un sel choisi dans le groupe consistant en des sels d'ammonium, de sulfonium, de sodium, de potassium, de strontium, de calcium et de magnésium.

**9.** Macromonomère estérifié suivant l'une quelconque des revendications 1 à 8, dans lequel l'estérification est effectuée dans un solvant choisi dans le groupe consistant en le THF, le bisméthacrylate de triéthylène-glycol, le bisméthacrylate de diéthylène-glycol, le bisméthacrylate de dioxolanne, un spiro-ortho-ester, spiro-orthocarbonate

ou bicyclo-ortho-ester à substituant vinyle, vinylène ou vinylidène, acrylate ou méthacrylate, et le 2,2-bis [p-(acryloxyéthoxy)phényl]propane.

**10.** Macromonomère estérifié suivant l'une quelconque des revendications 1 à 8, dans lequel ladite estérification est effectuée en présence d'une amine tertiaire.

**11.** Macromonomère estérifié suivant la revendication 1, dans lequel le nombre desdits groupements acides est choisi de manière à obtenir un polymère par polymérisation dudit monomère ayant une force d'adhérence à la dentine d'au moins 2 MPa.

**12.** Composition dentaire/médicale comprenant le macromonomère suivant la revendication 2, une charge, un monomère polymérisable ayant au moins un groupe ester de phosphore, un initiateur de polymérisation et un stabilisant.

**13.** Composition suivant la revendication 12, comprenant en outre un monomère polymérisable choisi dans le groupe consistant en des (méth)acrylates mono- et poly-fonctionnels, des di- et poly(méth)acrylates d'uréthanne, un spiro-ortho-ester, spiro-orthocarbonate ou bicyclo-ortho-ester à substituant vinyle, vinylène ou vinylidène, acrylate ou méthacrylate, ledit monomère étant présent en une proportion d'environ 5 à environ 80 % en poids.

**14.** Composition suivant la revendication 12, dans laquelle l'initiateur de polymérisation est un initiateur thermique, un initiateur redox ou un photo-initiateur.

**15.** Composition suivant la revendication 12, dans laquelle ladite charge comprend une charge inorganique et/ou une charge organique.

**16.** Composition suivant la revendication 15, dans laquelle ladite charge est une charge inorganique libérant des ions fluorure.

**17.** Composition formée en polymérisant la composition suivant la revendication 12 pour former un produit polymère ayant une adhérence à la dentine d'au moins 2 MPa, une libération d'ions fluorure d'au moins 1 µg de $F^-$ par semaine et par $cm^2$ de surface exposée de la composition, une opacité d'au moins $C_{0,7}$ = 40 % et une résistance à la compression d'au moins 200 MPa.

**18.** Composition suivant la revendication 12, comprenant environ 5 à environ 20 % en poids dudit macromonomère estérifié, environ 10 à environ 25 % en poids d'un monomère di- ou poly(méth)acrylate ayant au moins un groupe ester d'acide dérivé du phosphore, environ 20 à environ 35 % en poids d'un monomère polymérisable, environ 50 à environ 65 % en poids d'une charge et un initiateur de polymérisation et des stabilisants.

**19.** Composition suivant la revendication 12, comprenant environ 3 à environ 15 % en poids dudit macromonomère estérifié, environ 5 à environ 25 % en poids de monomère di- ou poly(méth)acrylate ayant au moins un groupe ester d'acide dérivé du phosphore, environ 7 à environ 40 % en poids d'un monomère polymérisable, environ 50 à environ 85 % en poids d'une charge et un initiateur de polymérisation et des stabilisants.

**20.** Composition suivant la revendication 12, comprenant environ 5 à environ 25 % en poids dudit macromonomère estérifié, environ 10 à environ 30 % en poids d'un monomère di- ou poly(méth)acrylate ayant au moins un groupe ester d'acide dérivé du phosphore, environ 20 à environ 40 % en poids d'un monomère polymérisable, environ 10 à environ 50 % en poids d'une charge et un initiateur de polymérisation et des stabilisants.

**21.** Composition suivant la revendication 12, comprenant environ 5 à environ 25 % en poids dudit macromonomère estérifié, environ 5 à environ 30 % en poids de monomère di- ou poly(méth)acrylate ayant au moins un groupe ester d'acide dérivé du phosphore, environ 40 % en poids d'un monomère polymérisable, environ 30 à environ 90 % en poids d'un diluant et un initiateur et des stabilisants.

**22.** Composition suivant la revendication 12, comprenant environ 1 à environ 25 % en poids dudit macromonomère estérifié, un monomère di- ou poly(méth)acrylate ayant au moins un groupe ester d'acide dérivé du phosphore et un monomère polymérisable et un initiateur de polymérisation, environ 75 à environ 99 % en poids d'un solvant organique et un co-initiateur de polymérisation.

## Patentansprüche

1. Verestertes Makromonomer, erhältlich durch Verestern mindestens eines Teiles der -OH-Gruppen eines OH-Gruppen enthaltenden Makromonomers, das mindestens eine endständige Doppelbindung aufweist, mit mindestens einem Derivat einer anorganischen oder organischen Saure, wodurch anhängende Gruppen eingeführt werden, die mindestens eine Säuregruppierung zeigen, ausgewählt aus der Gruppe bestehend aus -COOH, $-PO_3H_2$, $-SO_3H$, $-BO_2H$ oder deren Salzen, wodurch das veresterte Makromonomer im Rahmen der allgemeinen Formel liegt:

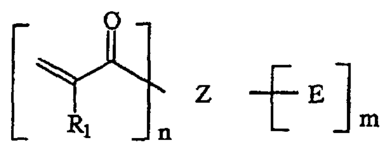

worin Z eine organische Gruppierung ist,

| | |
|---|---|
| $R_1$ | Wasserstoff oder ein substituiertes oder unsubstituiertes Alkyl mit 1 bis 12 Kohlenstoffatomen, Oxyalkyl mit 1 bis 12 Kohlenstoffatomen, Alkenyl mit 2 bis 12 Kohlenstoffatomen, Cycloalkyl mit 5 bis 12 Kohlenstoffatomen, Aryl mit 6 bis 12 Kohlenstoffatomen oder Aralkyl mit 7 bis 12 Kohlenstoffatomen ist, |
| jedes E | unabhängig eine Hydroxylgruppe oder eine organische oder anorganische Estergruppierung ist und mindestens ein E eine organische oder anorganische Estergruppierung ist, |
| n und m | jeweils unabhängig eine ganze Zahl von 2 bis 12 sind. |

2. Verestertes Makromonomer nach Anspruch 1, worin das veresterte Makromonomer im Rahmen mindestens einer der Formeln M-1 bis M-12 liegt:

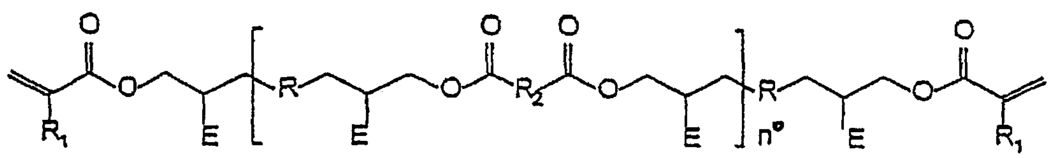

M-1

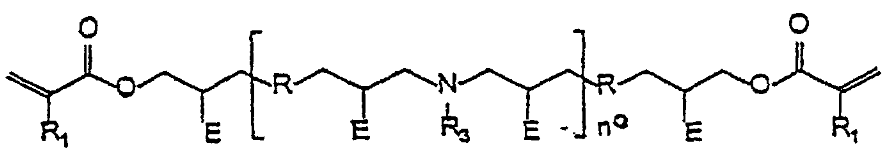

M-2

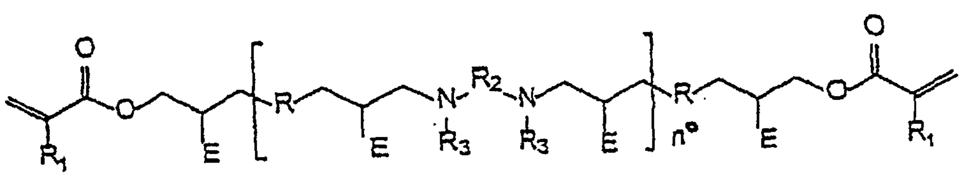

M-3

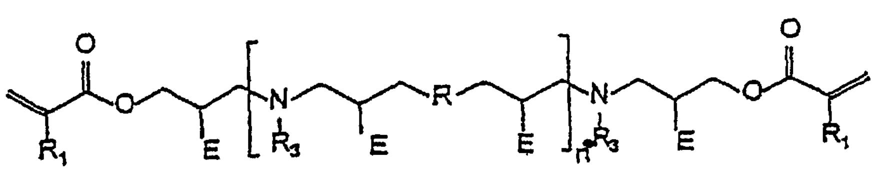

M-4

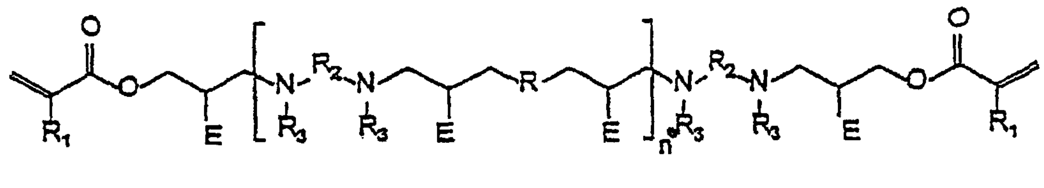

M-5

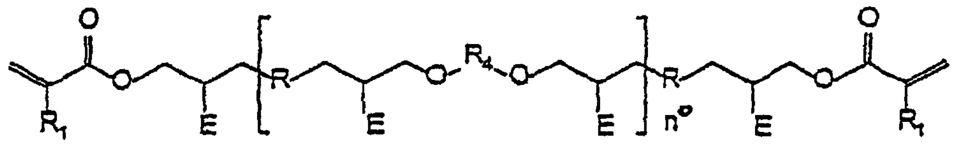

M-6

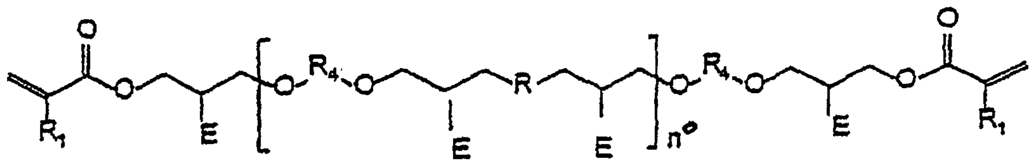

M-7

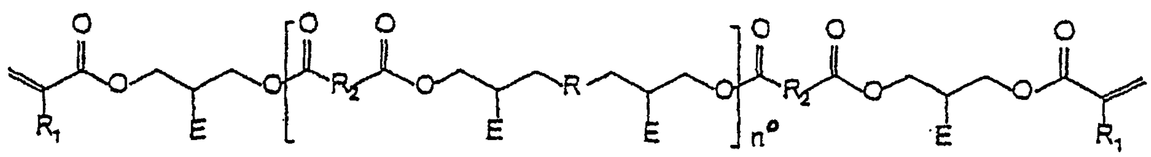

M-8

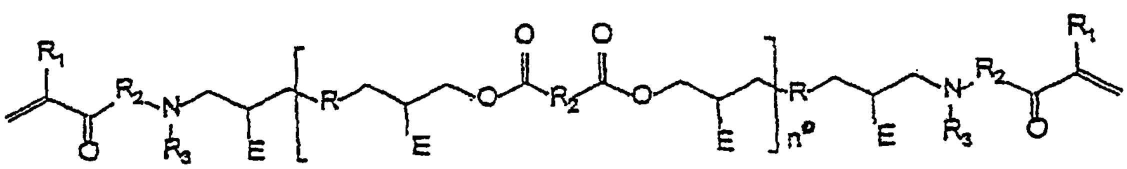

M-9

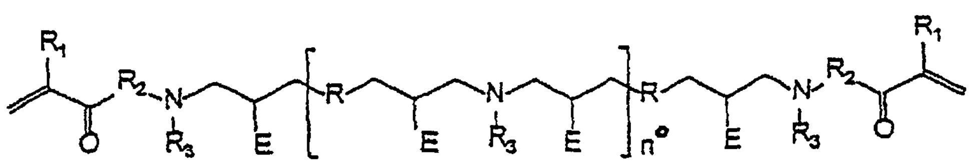

M-10

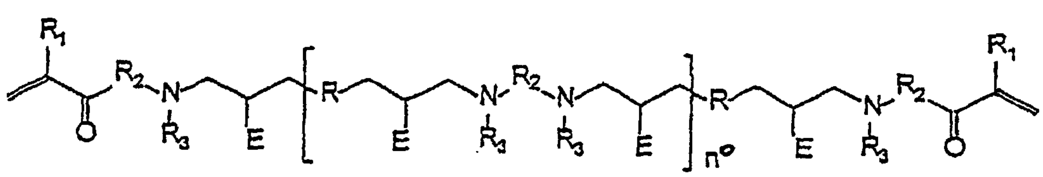

M-11

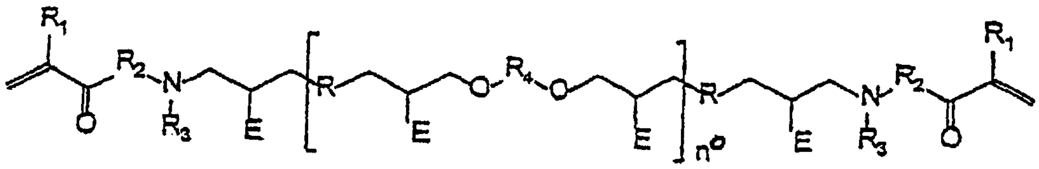 M-12

worin E und $R_1$ die in Anspruch 1 angegebenen Bedeutungen haben,

- R eine Diether enthaltende Gruppierung oder Diester enthaltende Gruppierung oder Tertiäramin enthaltende Gruppierung ist,
- $R_2$ eine difunktionelle substituierte oder unsubstituierte Alkylgruppe mit 1 bis 12 Kohlenstoffatomen, Alkenylgruppe mit 2 bis 12 Kohlenstoffatomen, Cycloalkyl mit 5 bis 12 Kohlenstoffatomen, Aryl mit 6 bis 12 Kohlenstoffatomen oder Aralkyl mit 7 bis 12 Kohlenstoffatomen ist,
- $R_3$ Wasserstoff oder eine substituierte oder unsubstituierte Alkylgruppe mit 1 bis 12 Kohlenstoffatomen, Alkenylgruppe mit 2 bis 12 Kohlenstoffatomen, Cycloalkyl mit 6 bis 12 Kohlenstoffatomen, Aryl mit 6 bis 12 Kohlenstoffatomen oder Aralkyl mit 7 bis 12 Kohlenstoffatomen ist,
- $R_4$ ein substituiertes oder unsubstituiertes Aryl mit 6 bis 12 Kohlenstoffatomen ist, und
- n° eine ganze Zahl von mindestens 1 ist.

**3.** Verestertes Makromonomer nach Anspruch 2, worin R im Rahmen der allgemeinen Formel liegt

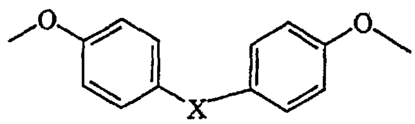 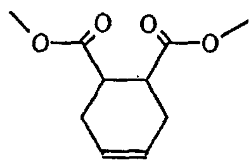

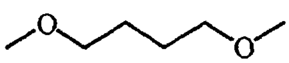 oder 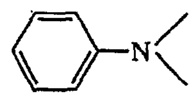

worin X >$C(CH_3)_2$, $-CH_2-$, -O-, -S-, -CO- oder $-SO_2-$ ist.

**4.** Verestertes Makromonomer nach Anspruch 2, worin R

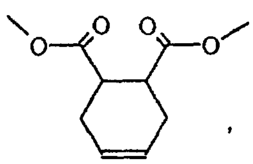 , 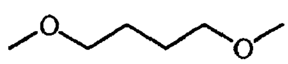 oder 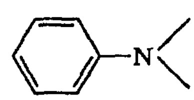

ist.

**5.** Verestertes Makromonomer nach Anspruch 2, worin $R_4$ im Rahmen mindestens einer der allgemeinen Formeln liegt:

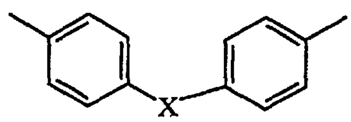

oder

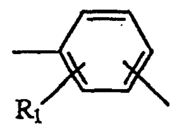

worin X >$C(CH_3)_2$, $-CH_2-$, -O-, -S-, -CO- oder $-SO_2-$ ist.

**6.** Verestertes Makromonomer nach Anspruch 2, worin mindestens ein E eine Carboxylgruppe umfasst.

**7.** Verestertes Makromonomer nach Anspruch 1, worin E abgeleitet ist von Bernsteinsäureanhydrid, Maleinsäureanhydrid, Dichlormaleinsäureanhydrid, Dimethylmaleinsäureanhydrid, Äpfelsäureanhydrid, Aconitsäureanhydrid, Adipinsäureanhydrid, 3,3-Tetramethylenglutarsäureanhydrid, Cyclohexen-1,2-säureanhydrid, Endomethylentetrahydrophthalsäureanhydrid (nadinic acid anhydride), Phthalsäureanhydrid, Trimellitsäureanhydrid, 2-Sulfobenzoesäureanhydrid, 2-Sulfobernsteinsäureanhydrid, Phthalsäureanhydrid-p-(O-phosphat), Phthaloylahlorid, Bernsteinsäuredimethylester, Phosphorpentachlorid, Phosphortrichlorid, Phosphoroxychlorid, Sulfurylchlorid, Thionylchlorid, Phosphorthionylchlorid, Borsäureanhydrid und Bortrichlorid.

**8.** Verestertes Makromonomer nach Anspruch 1, worin mindestens ein E ein Salz ist, ausgewählt aus der Gruppe bestehend aus Ammonium-, Sulfonium-, Natrium-, Kalium-, Strontium-, Calcium- und Magnesiumsalzen.

**9.** Verestertes Makromonomer nach einem der Ansprüche 1 bis 8, worin die Veresterung in einem Lösungsmittel ausgeführt wird, ausgewählt aus der Gruppe bestehend aus THF, Triethylenglykolbismethacrylat, Diethylenglykolbismethacrylat, Dioxolanbismethacrylat, Vinyl-, Vinylenoder Vinyliden-, Acrylat- oder Methacrylat-substituiertem Spiroorthoester, Spiroorthocarbonat oder Bicycloorthoester und 2,2.Bis[t-(acryloxyethoxy)phenyl]propan.

**10.** Verestertes Makromonomer nach einem der Ansprüche 1 bis 8, worin die Veresterung in Gegenwart eines tertiären Amins ausgeführt wird.

**11.** Verestertes Makromonomer nach Anspruch 1, worin die Anzahl der Säuregruppierungen derart ausgewählt ist, dass ein durch Polymerisieren des Monomers erhaltenes Polymer eine Klebefestigkeit an Dentin von mindestens 2 MPa aufweist.

**12.** Dentale/medizinische Zusammensetzung, umfassend das Makromonomer nach Anspruch 1, einen Füllstoff, ein polymerisierbares Monomer mit mindestens einer Phosphorestergruppe, einen Polymerisations-Initiator und einen Stabilisator.

**13.** Zusammensetzung nach Anspruch 12, weiter umfassend ein polymerisierbares Monomar, ausgewählt aus der Gruppe bestehend aus mono- und poly-funktionellem (Meth)acrylat, einem Urethan Di- und Poly(meth)acrylat, einem Vinyl-, Vinylen- oder Vinyliden-, Acrylat- oder Methacrylat-substituierten Spiroorthoester, einem Spiroorthocarbonat oder einem Bicycloorthoester, wobei das Monomer von etwa 5 bis etwa 80 Gew.-% umfasst.

**14.** Zusammensetzung nach Anspruch 12, worin der Polymerisations-Initiator ein thermischer Initiator, ein Redox-Initiator oder ein Fotoinitiator ist.

**15.** Zusammensetzung nach Anspruch 12, worin der Füllstoff einen anorganischen Füllstoff und/oder einen organischen Füllstoff umfasst.

**16.** Zusammensetzung nach Anspruch 15, worin der Füllstoff ein Fluorid freisetzender, anorganischer Füllstoff ist.

**17.** Zusammensetzung, gebildet durch Polymerisieren der Zusammensetzung nach Anspruch 12 zur Bildung eines polymeren Produktes mit einer Haftung an Dentin von mindestens 2 MPa, einer Fluorid-Abgabe von mindestens 1 $\mu g$ $F^-$ pro Woche und pro $cm^2$ der exponierten Oberfläche der Zusammensetzung, einer Opazität von mindestens $C_{0,7}$=40% und einer Kompressionsfestigkeit von mindestens 200 MPa.

**18.** Zusammensetzung nach Anspruch 12, umfassend von etwa 5 bis etwa 20 Gew.-% des veresterten Makromonomers, von etwa 10 bis etwa 25 Gew.-% eines Di- oder Poly(meth)acrylatmonomers mit mindestens einer Phosphorsäureestergruppe, von etwa 20 bis etwa 35 Gew.-% eines polymerisierbaren Monomers, von etwa 50 bis etwa 65 Gew.-% eines Füllstoffes und Polymerisations-Initiator und Stabilisatoren.

**19.** Zusammensetzung nach Anspruch 12, umfassend von etwa 3 bis etwa 15 Gew.-% des veresterten Makromonomers, von etwa 5 bis etwa 25 Gew.-% eines Di- oder Poly(meth)acrylatmonomers mit mindestens einer Phosphorsäureestergruppe, von etwa 7 bis etwa 40 Gew.-% eines polymerisierbaren Monomers, von etwa 50 bis etwa 85 Gew.-% eines Füllstoffes und Polymerisations-Initiator und Stabilisatoren.

**20.** Zusammensetzung nach Anspruch 12, umfassend von etwa 5 bis etwa 25 Gew.-% des veresterten Makromonomers, von etwa 10 bis etwa 30 Gew.-% eines Di- oder Poly(meth)acrylatmonomers mit mindestens einer Phosphorsäureestergruppe, von etwa 20 bis etwa 40 Gew.-% eines polymerisierbaren Monomers, von etwa 10 bis etwa 50 Gew.-% eines Füllstoffes und Polymerisations-Initiator und Stabilisatoren.

**21.** Zusammensetzung nach Anspruch 12, umfassend von etwa 5 bis etwa 25 Gew.-% des veresterten Makromonomers, von etwa 5 bis etwa 30 Gew.-% eines Di- oder Poly(meth)acrylatmonomers mit mindestens einer Phosphorsäureestergruppe, von etwa 40 Gew.-% eines polymerisierbaren Monomers, von etwa 30 bis etwa 90 Gew.-% eines Verdünnungsmittels und Initiator und Stabilisatoren.

**22.** Zusammensetzung nach Anspruch 12, umfassend etwa 1 bis etwa 25 Gew.-% des veresterten Makromonomers, Di- oder Poly(meth)acrylat-monomer mit mindestens einer Phosphorsäureestergruppe und einem polymerisierbaren Monomer und Polymerisations-Initiator, von etwa 75 bis etwa 99 Gew.-% eines organischen Lösungsmittels und Polymerisations-Coinitiator.